# EUROPEAN PATENT APPLICATION

(11) **EP 4 769 293 A1**
(43) Date of publication of application: **01.07.2026**
(21) Application number: 24856372.8
(22) Date of filing: 13.08.2024
(51) Int. Cl.: G06T 5/60, G01N 33/48, G06T 1/00, G06T 7/00

(54) **IMAGE PROCESSING DEVICE, OPERATION METHOD FOR IMAGE PROCESSING DEVICE, AND OPERATION PROGRAM FOR IMAGE PROCESSING DEVICE**

(30) Priority: 23.08.2023 JP 2023135822
(71) Applicant: FUJIFILM Corporation, Tokyo 106-8620 (JP)
(72) Inventor: TOMINAGA, Shunsuke, Ashigarakami-gun, Kanagawa 258-8577 (JP); INOMATA, Akira, Ashigarakami-gun, Kanagawa 258-8577 (JP)
(74) Representative: HGF
(86) International application number: PCT/JP2024/028918
(87) International publication number: WO 2025/041681

(57) **Abstract**

An image processing apparatus comprising a processor, in which the processor is configured to acquire a specimen image in which a tissue specimen of a subject provided for an evaluation test of a candidate substance of a drug is imaged, and perform image processing on the specimen image in accordance with a type of a source organ of the tissue specimen and/or a type of a morphological abnormality estimated to have occurred in the tissue specimen.

## Description

### BACKGROUND OF THE INVENTION

### 1. Field of the Invention

The technology of the present disclosure relates to an image processing apparatus, an operation method of an image processing apparatus, and an operation program of an image processing apparatus.

### 2. Description of the Related Art

In a process of pharmaceutical development, a test is performed in which a candidate substance of a drug is administered to a subject such as a rat, and drug efficacy and toxicity of the candidate substance are evaluated. In such an evaluation test, a specimen image in which a tissue specimen (a brain specimen, a liver specimen, a heart specimen, or the like) of an organ collected by performing an autopsy on the subject is imaged is used. The specimen image is digitized to be a whole slide image (WSI). The specimen image is displayed on a display of a computer and is provided for browsing by a user such as a pathologist who is responsible for evaluating the candidate substance.

In the related art, various techniques have been proposed in which image processing is performed on a specimen image to support the evaluation of the candidate substance by the user. For example, JP6780045B discloses a technique in which a computer evaluates a quality of a specimen image and performs image processing such as contrast correction, color correction, and sharpening on the specimen image having a low quality. In addition, JP5996494B discloses a technique in which, as a display time of a region of interest of the user in the specimen image increases, the resolution of the region of interest is displayed in a stepwise manner, and in a case where the highest resolution is reached, image processing is performed to make specific staining of the region of interest more noticeable.

### SUMMARY OF THE INVENTION

There are many types of organs serving as sources of the tissue specimens, such as a brain, an esophagus, a stomach, a large intestine, a small intestine, a liver, a kidney, a spleen, a pancreas, a heart, a testis or an ovary, a lymph node, and a bone marrow. In addition, in a case where the candidate substance is evaluated, the user observes a region in which a morphological abnormality (a lesion that is not observed in a normal tissue specimen) is estimated to have occurred in the tissue specimen in a focused manner, but there are many types of morphological abnormalities, such as hyperplasia, infiltration, congestion, cyst, inflammation, tumor, carcinogenesis, proliferation, bleeding, glycogen reduction, inclusion body, granular cytoplasm, and foamy cytoplasm.

In order to accurately evaluate the candidate substance using the specimen image in which the tissue specimen of the plurality of types of organs and the tissue specimen including the plurality of types of morphological abnormalities are imaged, it is necessary to perform suitable image processing on the specimen image. However, the image processing disclosed in JP6780045B and JP5996494B may not necessarily be suitable image processing. Therefore, there is a concern that the accuracy of the evaluation of the candidate substance may be reduced.

One embodiment according to the technology of the present disclosure provides an image processing apparatus, an operation method of an image processing apparatus, and an operation program of an image processing apparatus, which can suppress a decrease in accuracy of the evaluation of the candidate substance.

An image processing apparatus according to the present disclosure comprises a processor, in which the processor is configured to acquire a specimen image in which a tissue specimen of a subject provided for an evaluation test of a candidate substance of a drug is imaged, and perform image processing on the specimen image in accordance with a type of a source organ of the tissue specimen and/or a type of a morphological abnormality estimated to have occurred in the tissue specimen.

It is preferable that the image processing is image processing that simulates a change in image quality of the specimen image due to a change in setting of an optical system in an imaging apparatus.

It is preferable that the image processing includes at least one of processing of adjusting brightness, processing of adjusting contrast, or enlargement processing.

It is preferable that the processor is configured to perform the image processing only on a region in which the morphological abnormality is estimated to have occurred.

It is preferable that the processor is configured to determine the type of the source organ of the tissue specimen and/or the type of the morphological abnormality.

It is preferable that the processor is configured to receive an instruction to perform the image processing from a user.

It is preferable that the processor is configured to extract a region in which the morphological abnormality is estimated to have occurred from the specimen image.

It is preferable that the processor is configured to perform the extraction by using a machine learning model.

It is preferable that the processor is configured to perform the extraction by comparing a feature amount obtained by inputting the specimen image to the machine learning model with a reference feature amount obtained by inputting a reference specimen image in which a tissue specimen considered to be normal is imaged to the machine learning model.

It is preferable that the processor is configured to classify the region in which the morphological abnormality is estimated to have occurred into a plurality of groups based on similarity of the morphological abnormality, and perform the same image processing on a group basis.

An operation method of an image processing apparatus according to the present disclosure comprises acquiring a specimen image in which a tissue specimen of a subject provided for an evaluation test of a candidate substance of a drug is imaged, and performing image processing on the specimen image in accordance with a type of a source organ of the tissue specimen and/or a type of a morphological abnormality estimated to have occurred in the tissue specimen.

An operation program of an image processing apparatus according to the present disclosure causes a computer to execute a process comprising acquiring a specimen image in which a tissue specimen of a subject provided for an evaluation test of a candidate substance of a drug is imaged, and performing image processing on the specimen image in accordance with a type of a source organ of the tissue specimen and/or a type of a morphological abnormality estimated to have occurred in the tissue specimen.

According to the technology of the present disclosure, it is possible to provide an image processing apparatus, an operation method of an image processing apparatus, and an operation program of an image processing apparatus, which can suppress a decrease in accuracy of the evaluation of the candidate substance.

### BRIEF DESCRIPTION OF THE DRAWINGS

FIG. 1 is a diagram showing a generation procedure of a specimen image, the specimen image, and an evaluation support apparatus.
FIG. 2 is a diagram showing a dose group and a control group.
FIG. 3 is a block diagram showing a computer constituting the evaluation support apparatus.
FIG. 4 is a block diagram showing a processing unit of a CPU of the evaluation support apparatus.
FIG. 5 is a diagram showing processing of an identification unit.
FIG. 6 is a diagram showing processing of a first determination unit.
FIG. 7 is a diagram showing processing of the first determination unit.
FIG. 8 is a diagram showing an extraction model group.
FIG. 9 is a diagram showing a patch image obtained by subdividing a region image.
FIG. 10 is a diagram showing a state in which a feature amount is extracted from the patch image by an extraction model.
FIG. 11 is a diagram showing a configuration of the extraction model.
FIG. 12 is a diagram showing a process in a learning phase of an autoencoder.
FIG. 13 is a diagram showing a configuration of a past control group and a reference patch image for learning.
FIG. 14 is a diagram showing a state in which a reference feature amount is extracted from a reference patch image by the extraction model.
FIG. 15 is a diagram showing a graph in which the reference feature amount is plotted in a feature amount space and extraction reference information.
FIG. 16 is a diagram showing a distance between a position of the feature amount and a representative position of the reference feature amount.
FIG. 17 is a diagram showing processing of an extraction unit and a determination result.
FIG. 18 is a diagram showing processing of the extraction unit and a determination result.
FIG. 19 is a diagram showing processing of a second determination unit.
FIG. 20 is a diagram showing image processing information.
FIG. 21 is a diagram showing image processing.
FIG. 22 is a diagram showing image processing.
FIG. 23 is a diagram showing an image list display screen.
FIG. 24 is a diagram showing a first image display screen.
FIG. 25 is a diagram showing a state in which a frame indicating a morphological abnormality occurrence estimation region is displayed on the first image display screen.
FIG. 26 is a diagram showing a second image display screen.
FIG. 27 is a flowchart showing a processing procedure of the evaluation support apparatus.
FIG. 28 is a flowchart showing a processing procedure of the evaluation support apparatus.
FIG. 29 is a flowchart showing a processing procedure of the evaluation support apparatus.
FIG. 30 is a flowchart showing a processing procedure of the evaluation support apparatus.
FIG. 31 is a flowchart showing a processing procedure of the evaluation support apparatus.
FIG. 32 is a diagram showing a first image display screen of a second embodiment.
FIG. 33 is a diagram showing a second image display screen of the second embodiment.
FIG. 34 is a diagram showing clustering processing.
FIG. 35 is a diagram showing a display format of each cluster, a cluster image, and a superimposition image.
FIG. 36 is a diagram showing a first image display screen of a third embodiment.
FIG. 37 is a diagram showing image processing of the third embodiment.

### DESCRIPTION OF THE PREFERRED EMBODIMENTS

### [First Embodiment]

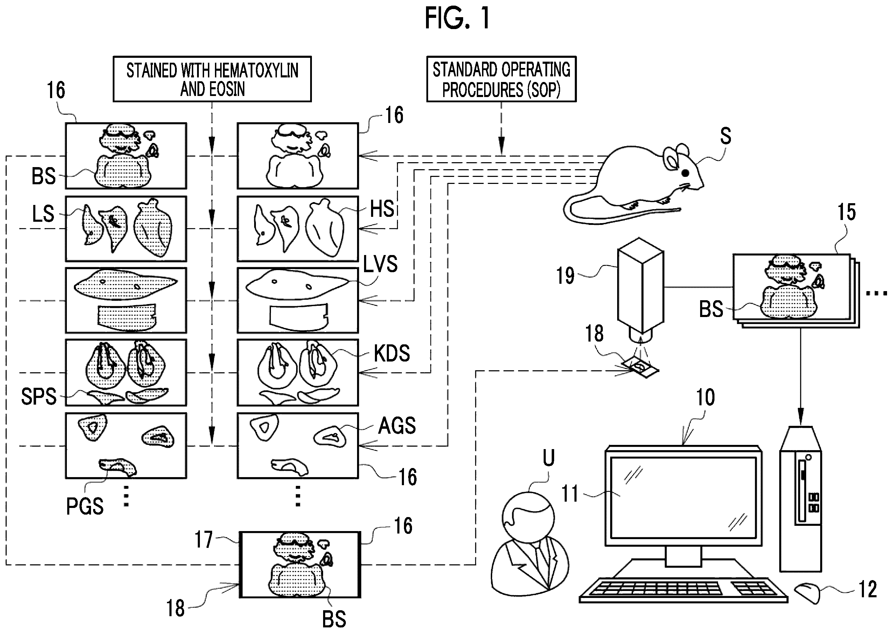

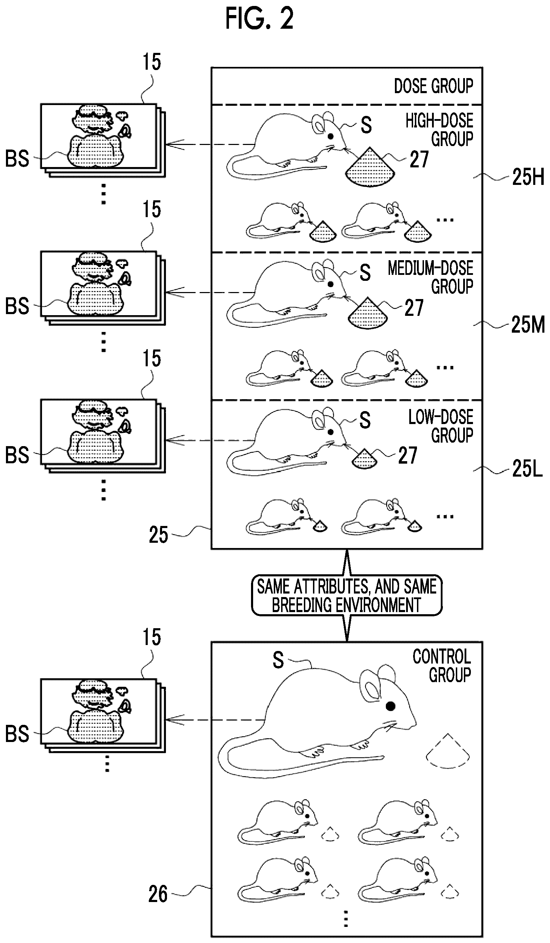

As shown in FIG. 1 as an example, an evaluation support apparatus 10 is used for evaluating drug efficacy and toxicity of a candidate substance 27 (see FIG. 2) of a drug. The evaluation support apparatus 10 is an example of an "image processing apparatus" according to the technology of the present disclosure. The drug is, for example, a biopharmaceutical such as an antibody pharmaceutical having an antibody as an active ingredient, or a peptide pharmaceutical, a nucleic acid pharmaceutical, or the like having a peptide or a nucleic acid as an active ingredient.

The evaluation support apparatus 10 is, for example, a desktop personal computer and comprises a display 11 that displays various screens and an input device 12 such as a keyboard, a mouse, a touch panel, and/or a microphone for voice input. The evaluation support apparatus 10 is installed in, for example, a pharmaceutical company that develops a drug or an institution that receives a development business of the drug from the pharmaceutical company, that is, a contract research organization (CRO). The evaluation support apparatus 10 is operated by a user U who is involved in the development of the drug in a pharmaceutical company or a contract research organization (hereinafter, collectively referred to as a pharmaceutical facility). The user U is, for example, a pathologist who is responsible for evaluating the candidate substance 27.

A plurality of specimen images 15 are input to the evaluation support apparatus 10. The specimen image 15 is an image for evaluating the drug efficacy and the toxicity of the candidate substance 27. The specimen image 15 is generated, for example, by the following procedure. First, a subject S such as a rat prepared for the evaluation of the candidate substance 27 is autopsied, and a tissue specimen obtained by slicing an organ of the subject S is collected. The tissue specimen includes a brain specimen BS, a heart specimen HS, a lung specimen LS, a liver specimen LVS, a kidney specimen KDS, a spleen specimen SPS, an adrenal gland specimen AGS, a pituitary gland specimen PGS, and the like. Although not shown in FIG. 1, the tissue specimen also includes specimens of various organs such as an esophagus, a stomach, a large intestine, a small intestine, a pancreas, a gallbladder, an aorta, a large vein, a lymph node, a trachea, a bronchus, a diaphragm, a pineal gland, a testis or an ovary, and a spinal cord.

After the collection of the tissue specimen, each tissue specimen is attached to a slide glass 16 in accordance with standard operating procedures (SOP) predetermined for each pharmaceutical facility. The standard operating procedures describe, for example, designation of a fine layout of the tissue specimen, such as attaching the heart specimen HS and the lung specimen LS to the same slide glass 16 side by side. In this way, a plurality of tissue specimens are attached to one slide glass 16.

Thereafter, the tissue specimen is stained, here stained with hematoxylin and eosin dye. Subsequently, the stained tissue specimen is covered with a cover glass 17 to complete a slide specimen 18. Then, the slide specimen 18 is set in an imaging apparatus 19, such as a digital optical microscope, and the specimen image 15 is captured by the imaging apparatus 19. In the specimen image 15 obtained in this way, the entire tissue specimen attached to the slide glass 16 is imaged. In other words, a plurality of tissue specimens are imaged in one specimen image 15. The specimen image 15 is referred to as a whole slide image (WSI). The specimen image 15 is assigned with a subject identification data (ID) for uniquely identifying the subject S, a specimen image ID for uniquely identifying the specimen image 15, an imaging date and time, and the like. The tissue specimen is also referred to as a tissue section. In addition, the staining may be staining with a hematoxylin dye alone, staining with a nuclear fast red dye, or the like.

As shown in FIG. 2 as an example, the subject S is divided into a dose group 25 and a control group 26. The dose group 25 is composed of a plurality of subjects S to which the candidate substance 27 is administered. The dose group 25 is further divided into a high-dose group 25H, a medium-dose group 25M, and a low-dose group 25L according to the dose of the candidate substance 27. By dividing the dose group 25 into the high-dose group 25H, the medium-dose group 25M, and the low-dose group 25L in this way, it is possible to determine the influence on the subject S according to the dose of the candidate substance 27. The dose group 25 is not limited to being divided into the three groups of the high-dose group 25H, the medium-dose group 25M, and the low-dose group 25L illustrated as an example, and the dose group 25 may be divided into two groups of the high-dose group 25H and the low-dose group 25L, or the dose group 25 may be divided into four or more groups. In addition, the dose group 25 may be divided according to the length of the dosing period of the candidate substance 27. Alternatively, the dose group 25 may be divided according to the dosing frequency of the candidate substance 27.

The control group 26 is composed of a plurality of subjects S to which the candidate substance 27 is not administered, unlike the dose group 25. The number of subjects S constituting each of the high-dose group 25H, the medium-dose group 25M, and the low-dose group 25L and the number of subjects S constituting the control group 26 are the same, for example, about 5 to 10. The subject S constituting each of the high-dose group 25H, the medium-dose group 25M, and the low-dose group 25L and the subject S constituting the control group 26 are subjects S having the same attributes and placed in the same breeding environment. The same attributes include, for example, the same weekly age and/or the same gender. In addition, the same attributes also include the same weekly age composition ratio and/or the same gender composition ratio (for example, five males and five females). The same breeding environment means, for example, that feed is the same, that the temperature and humidity of a breeding space are the same, and/or that the size of the breeding space is the same. The "same" in the same breeding environment indicates not only the exact same, but also the same including an error that is generally allowed in the technical field to which the technology of the present disclosure belongs and that does not go against the gist of the technology of the present disclosure.

Since a plurality of specimen images 15 are obtained from one subject S, the number of specimen images 15 obtained from each group is obtained by multiplying the number of specimen images 15 obtained from one subject S by the number of subjects S. For example, in a case where the number of specimen images 15 obtained from one subject S is 100 and the number of subjects S constituting each group is 10, 100 × 10 = 1000 specimen images 15 are obtained from each group.

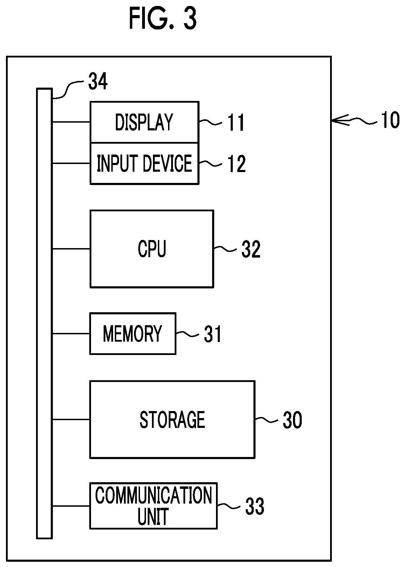

As shown in FIG. 3 as an example, a computer constituting the evaluation support apparatus 10 comprises a storage 30, a memory 31, a central processing unit (CPU) 32, and a communication unit 33 in addition to the display 11 and the input device 12 described above. These are connected to each other via a busline 34.

The storage 30 is a hard disk drive that is built into the computer constituting the evaluation support apparatus 10 or that is connected via a cable or a network. Alternatively, the storage 30 is a disk array in which a plurality of hard disk drives are connected in series. The storage 30 stores a control program, such as an operating system, various application programs, various types of data associated with these programs, and the like. A solid state drive may be used instead of the hard disk drive.

The memory 31 is a work memory for the CPU 32 to execute processing. The CPU 32 loads the program stored in the storage 30 into the memory 31 and executes the processing in accordance with the program. Therefore, the CPU 32 comprehensively controls each unit of the computer. In addition, the CPU 32 is an example of a "processor" according to the technology of the present disclosure. The memory 31 may be built into the CPU 32. The communication unit 33 controls the transmission of various types of information to an external device such as the imaging apparatus 19.

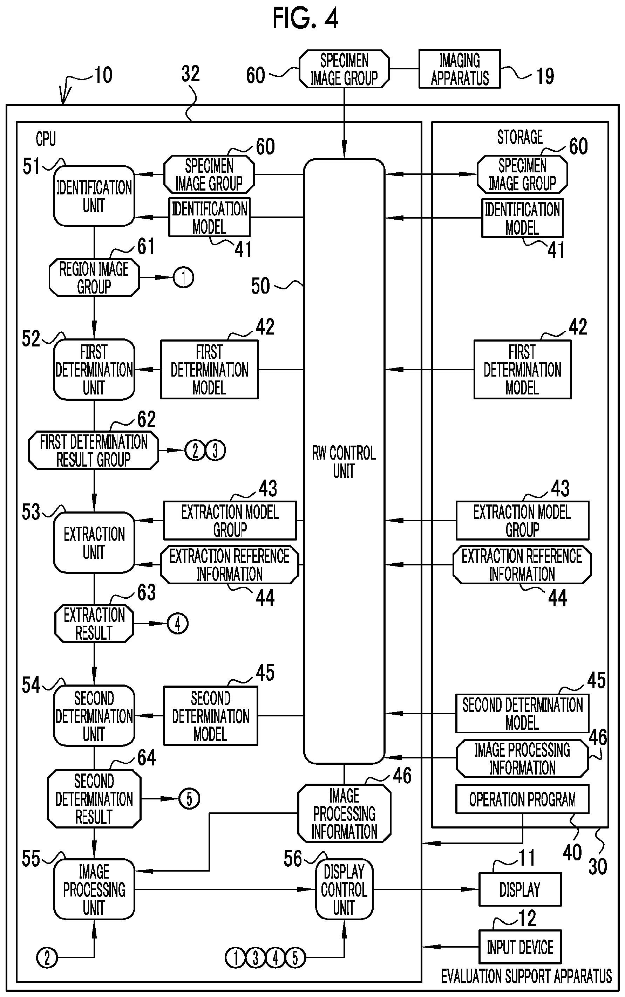

As shown in FIG. 4 as an example, an operation program 40 is stored in the storage 30 of the evaluation support apparatus 10. The operation program 40 is an application program for causing the computer to function as the evaluation support apparatus 10. That is, the operation program 40 is an example of an "operation program of an image processing apparatus" according to the technology of the present disclosure. The storage 30 also stores an identification model 41, a first determination model 42, an extraction model group 43, extraction reference information 44, a second determination model 45, image processing information 46, and the like.

In a case where the operation program 40 is activated, the CPU 32 of the computer constituting the evaluation support apparatus 10 functions as a read/write (hereinafter, abbreviated as RW) control unit 50, an identification unit 51, a first determination unit 52, an extraction unit 53, a second determination unit 54, an image processing unit 55, and a display control unit 56 in cooperation with the memory 31 and the like. In addition to each of the processing units 50 to 56, an instruction reception unit that receives various operation instructions from the input device 12 is also constructed in the CPU 32.

The RW control unit 50 controls the storage of various types of data in the storage 30 and the reading-out of various types of data in the storage 30. For example, the RW control unit 50 acquires a specimen image group 60 from the imaging apparatus 19 and stores the specimen image group 60 in the storage 30. The specimen image group 60 is a set of a plurality of specimen images 15 generated for evaluating the candidate substance 27.

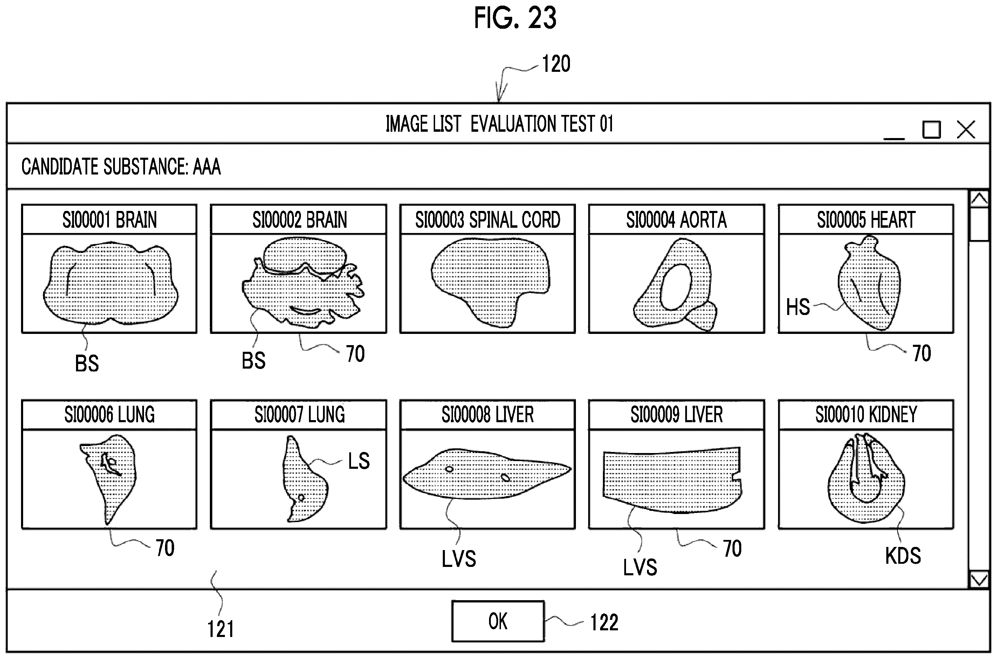

In a case where a display instruction of an image list display screen 120 (see FIG. 23) is issued by the user U through the input device 12, the RW control unit 50 reads out the specimen image group 60 from the storage 30. The RW control unit 50 outputs the read-out specimen image group 60 to the identification unit 51.

The RW control unit 50 reads out the identification model 41 from the storage 30 and outputs the read-out identification model 41 to the identification unit 51. In addition, the RW control unit 50 reads out the first determination model 42 from the storage 30 and outputs the read-out first determination model 42 to the first determination unit 52. Further, the RW control unit 50 reads out the extraction model group 43 and the extraction reference information 44 from the storage 30 and outputs the read-out extraction model group 43 and extraction reference information 44 to the extraction unit 53.

The RW control unit 50 reads out the second determination model 45 from the storage 30 and outputs the read-out second determination model 45 to the second determination unit 54. In addition, the RW control unit 50 reads out the image processing information 46 from the storage 30 and outputs the read-out image processing information 46 to the image processing unit 55.

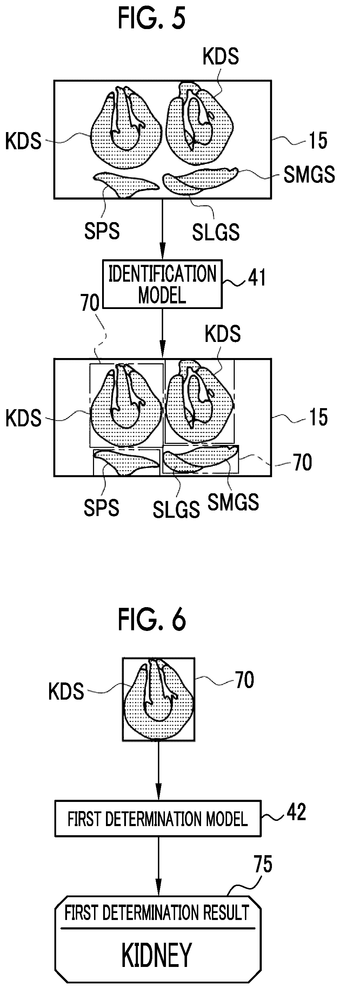

The identification unit 51 identifies the plurality of tissue specimens imaged in one specimen image 15 by using the identification model 41. Then, region images 70 (see FIG. 5) of the identified tissue specimens are generated from the specimen image 15. The identification unit 51 outputs a region image group 61, which is a set of the generated plurality of region images 70, to the first determination unit 52 and the display control unit 56.

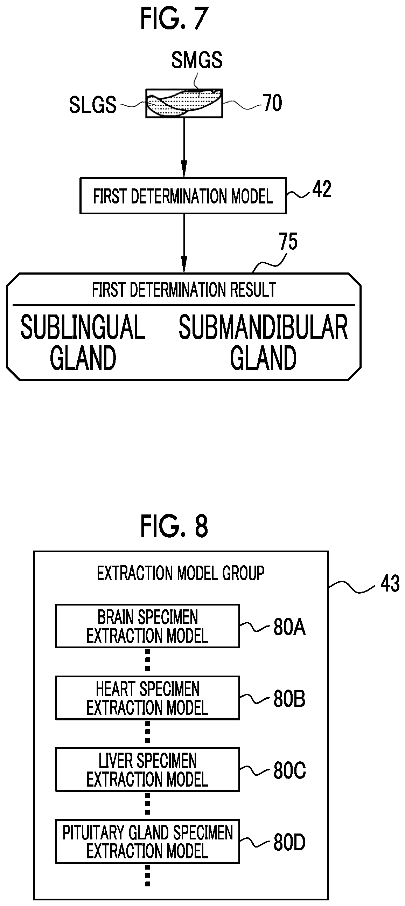

The first determination unit 52 determines the type of the organ of the tissue specimen imaged in the region image 70 by using the first determination model 42. Then, a first determination result group 62, which is a set of the region image 70 and a first determination result 75 (see FIGS. 6 and 7) of the type of the organ, is output to the extraction unit 53, the image processing unit 55, and the display control unit 56.

The extraction unit 53 extracts a region (hereinafter, referred to as a morphological abnormality occurrence estimation region) in which the morphological abnormality is estimated to have occurred in the tissue specimen imaged in the region image 70 by using the extraction model group 43 and the extraction reference information 44. Then, an extraction result 63 of the morphological abnormality occurrence estimation region is output to the second determination unit 54 and the display control unit 56. The morphological abnormality is a lesion that is not observed in a normal tissue specimen, for example, hyperplasia, infiltration, congestion, cyst, inflammation, tumor, carcinogenesis, proliferation, bleeding, glycogen reduction, inclusion body, granular cytoplasm, and foamy cytoplasm.

The second determination unit 54 determines the type of the morphological abnormality estimated to have occurred in the tissue specimen imaged in the region image 70 by using the second determination model 45. Then, a second determination result 64 of the type of the morphological abnormality is output to the image processing unit 55 and the display control unit 56.

The image processing unit 55 performs image processing on the region image 70 according to the image processing information 46, the first determination result 75, and the second determination result 64. The image processing unit 55 outputs the region image 70 after the image processing to the display control unit 56.

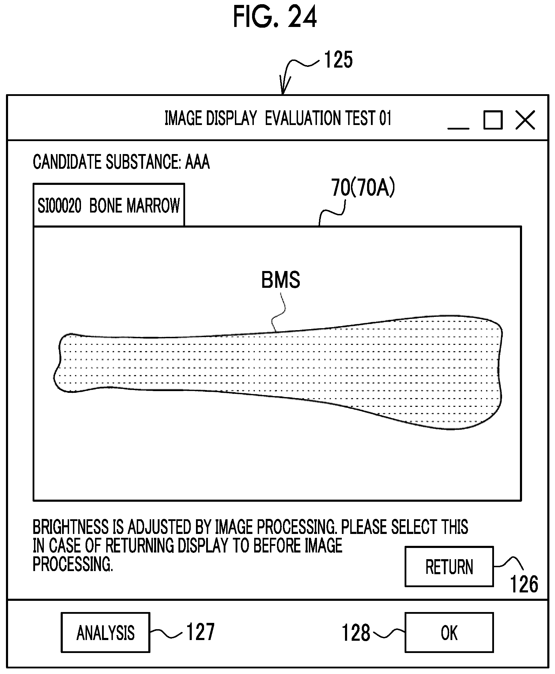

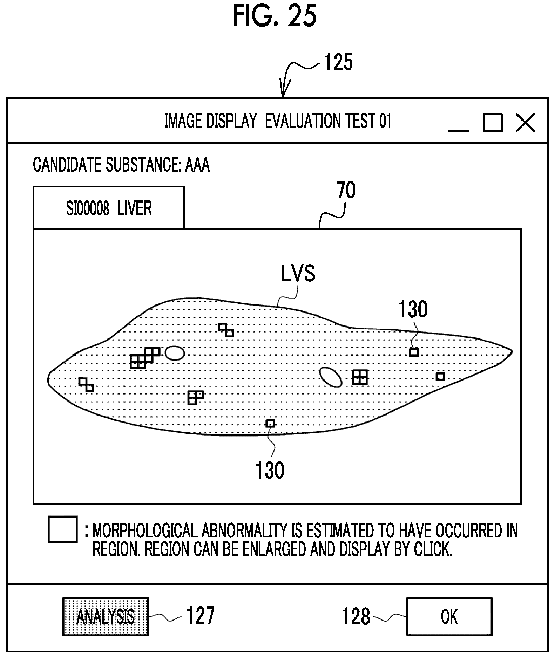

The display control unit 56 performs control of displaying various screens on the display 11. Various screens include an image list display screen 120 on which the region image 70 is displayed in a list, a first image display screen 125 (see FIGS. 24 and 25) on which each region image 70 is displayed, and the like.

As shown in FIG. 5 as an example, the identification unit 51 inputs the specimen image 15 to the identification model 41. Then, the identification model 41 identifies the plurality of tissue specimens imaged in the specimen image 15. The identification model 41 is, for example, a machine learning model such as a convolutional neural network. The identification model 41 identifies each of the plurality of tissue specimens imaged in the specimen image 15 one by one and outputs position coordinates of a rectangular frame (referred to as a bounding box) surrounding the tissue specimen as an identification result. The identification unit 51 generates the region image 70 of each identified tissue specimen by cutting out the rectangular frame from the specimen image 15 according to the identification result output by the identification model 41. A region image ID for uniquely identifying the region image 70 is assigned to the region image 70, as in the specimen image 15.

FIG. 5 illustrates the specimen image 15 in which two kidney specimens KDS, a spleen specimen SPS, a sublingual gland specimen SLGS, and a submandibular gland specimen SMGS are imaged. As can be seen from the example of the sublingual gland specimen SLGS and the submandibular gland specimen SMGS, a plurality of tissue specimens of organs may be mixed in the region image 70.

The rectangular frame surrounding the tissue specimen, which is the identification result of the identification model 41, may be configured to be modifiable by the user U. In addition, the tissue specimen imaged in the specimen image 15 may be identified by template matching instead of the identification model 41. Alternatively, the tissue specimen imaged in the specimen image 15 may be identified by inputting the rectangular frame surrounding the tissue specimen by hand of the user U without using the identification model 41 or the template matching.

As shown in FIGS. 6 and 7 as an example, the first determination unit 52 inputs the region image 70 to the first determination model 42. Then, the first determination model 42 determines the type of the organ of the tissue specimen imaged in the region image 70. The first determination model 42 is also, for example, a machine learning model such as a convolutional neural network, as in the identification model 41. The first determination model 42 outputs the first determination result 75 of the type of the organ of the tissue specimen imaged in the region image 70.

FIG. 6 illustrates a case where the region image 70 in which the kidney specimen KDS is imaged is input to the first determination model 42, and the first determination result 75 of "kidney" is output from the first determination model 42. In addition, FIG. 7 illustrates a case where the region image 70 in which the sublingual gland specimen SLGS and the submandibular gland specimen SMGS are imaged is input to the first determination model 42, and the first determination result 75 of "sublingual gland and submandibular gland" is output from the first determination model 42.

The first determination result 75 may be configured to be modifiable by the user U, or the determination of the type of the organ of the tissue specimen imaged in the region image 70 may be entrusted to the hand of the user U. In addition, the type of the organ of the tissue specimen imaged in the region image 70 may be determined by template matching instead of the first determination model 42.

As shown in FIG. 8 as an example, the extraction model group 43 is a set of extraction models 80 prepared for each organ. That is, the extraction model group 43 includes a brain specimen extraction model 80A used in the region image 70 in which the brain specimen BS is imaged, a heart specimen extraction model 80B used in the region image 70 in which the heart specimen HS is imaged, a liver specimen extraction model 80C used in the region image 70 in which the liver specimen LVS is imaged, a pituitary gland specimen extraction model 80D used in the region image 70 in which the pituitary gland specimen PGS is imaged, and the like. The extraction unit 53 uses the extraction model 80 in accordance with the first determination result 75. The extraction model 80 is an example of a "machine learning model" according to the technology of the present disclosure.

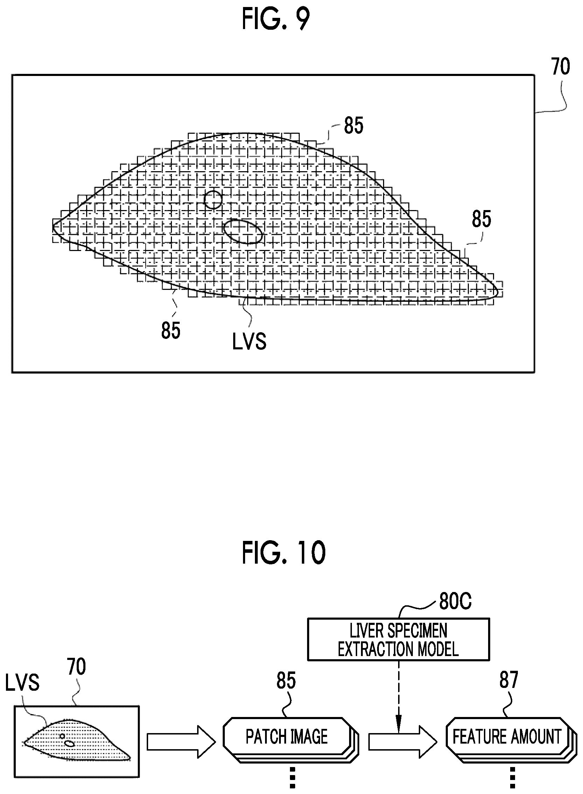

As shown in FIG. 9 as an example, the extraction unit 53 recognizes the tissue specimen (in FIG. 9, the liver specimen LVS is illustrated) imaged in the region image 70 by using a known image recognition technique, and subdivides the recognized tissue specimen into a plurality of patch images 85. The patch image 85 has a preset size that can be handled by the extraction model 80 (in this case, the liver specimen extraction model 80C). The extraction unit 53 assigns a patch image ID to the patch image 85. In addition, the extraction unit 53 associates the patch image ID with information indicating which position of the region image 70 is cut out by the patch image 85, that is, the position information of the patch image 85. In FIG. 9, the patch image 85 does not have a region that overlaps other patch images 85, but the patch image 85 may partially overlap other patch images 85.

As shown in FIG. 10 as an example, the extraction unit 53 extracts a feature amount 87 for each of the plurality of patch images 85 obtained by subdividing the region image 70 by using the extraction model 80 (in FIG. 10, the liver specimen extraction model 80C). Therefore, the number of feature amounts 87 is the same as the number of patch images 85.

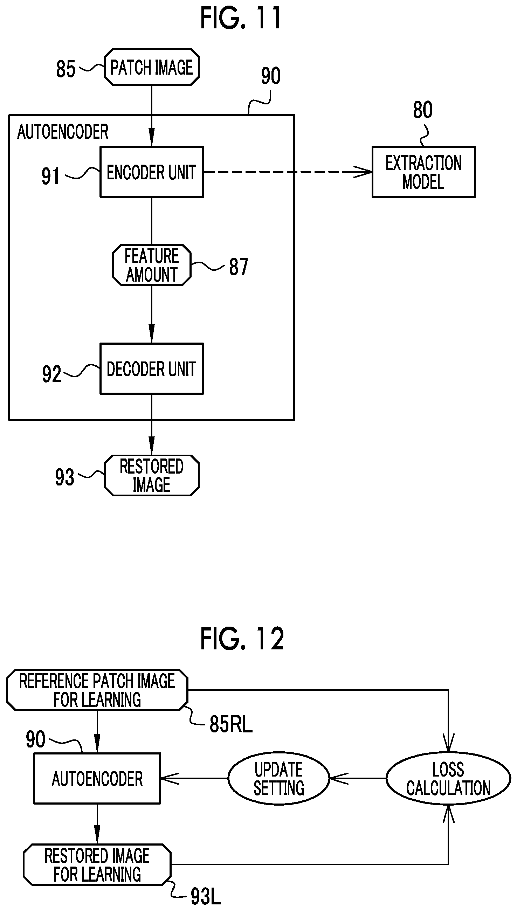

As shown in FIG. 11 as an example, an encoder unit 91 of an autoencoder 90 is used as the extraction model 80. The autoencoder 90 includes a decoder unit 92 in addition to the encoder unit 91. The patch image 85 is input to the encoder unit 91. The encoder unit 91 converts the patch image 85 into the feature amount 87. The encoder unit 91 delivers the feature amount 87 to the decoder unit 92. The decoder unit 92 generates a restored image 93 of the patch image 85 from the feature amount 87.

As is well known, the encoder unit 91 includes a convolutional layer that performs convolution processing using a filter, a pooling layer that performs pooling processing such as maximum value pooling processing, and the like. The same applies to the decoder unit 92. The encoder unit 91 repeatedly performs the convolution processing using the convolutional layer and the pooling processing using the pooling layer on the input patch image 85 a plurality of times to extract the feature amount 87. The extracted feature amount 87 represents a feature of a shape and a texture of the tissue specimen imaged in the patch image 85.

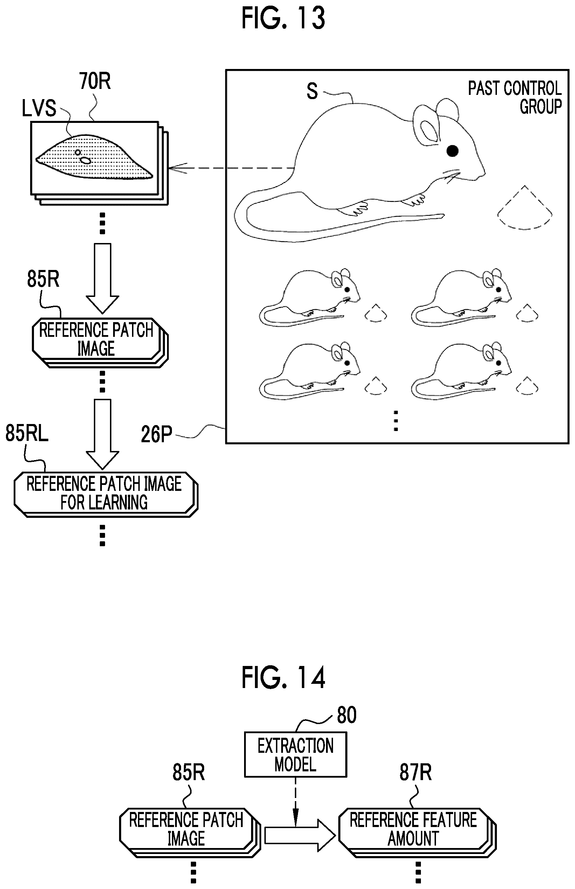

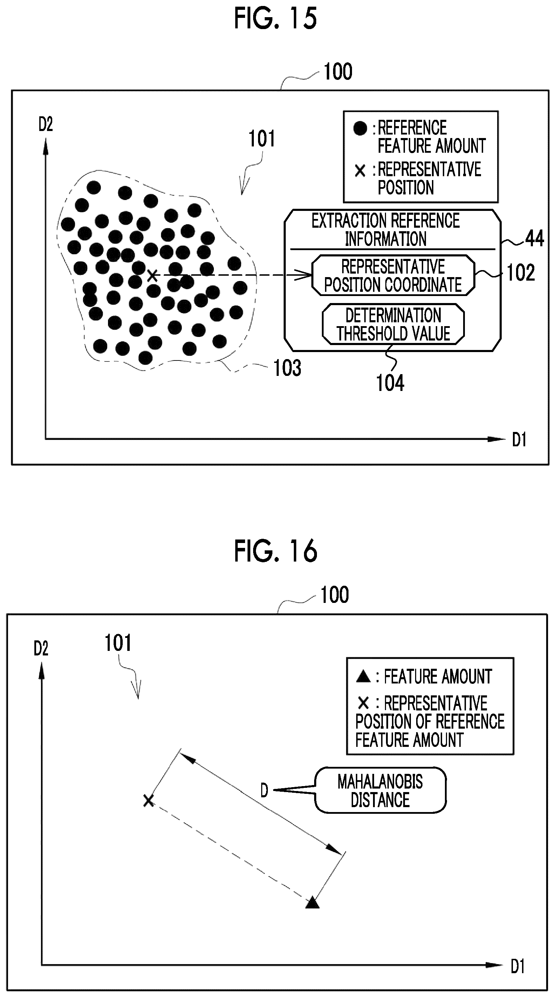

The feature amount 87 is a set of a plurality of numerical values. That is, the feature amount 87 is multi-dimensional data. The number of dimensions of the feature amount 87 is, for example, 512, 1024, or 2048. The feature amount 87 and a reference feature amount 87R (see FIG. 14) described below have the same number of dimensions and can be compared in the same feature amount space 101 (see FIG. 15 and the like).

As shown in FIG. 12 as an example, the autoencoder 90 is trained by inputting a reference patch image 85RL for learning in a learning phase before the encoder unit 91 is used as the extraction model 80. The autoencoder 90 outputs a restored image 93L for learning in response to the input of the reference patch image 85RL for learning. The loss calculation of the autoencoder 90 using a loss function is performed based on the reference patch image 85RL for learning and the restored image 93L for learning. Then, the update settings of various coefficients (for example, coefficients of convolutional layer filters) of the autoencoder 90 are performed according to the results of the loss calculation, and the autoencoder 90 is updated according to the update setting.

In the learning phase of the autoencoder 90, the series of processes of the input of the reference patch image 85RL for learning to the autoencoder 90, the output of the restored image 93L for learning from the autoencoder 90, the loss calculation, the update setting, and the update of the autoencoder 90 is repeatedly performed while the reference patch image 85RL for learning is exchanged. The repetition of the series of processes is ended in a case where the restoration accuracy from the reference patch image 85RL for learning to the restored image 93L for learning reaches a predetermined setting level. The encoder unit 91 of the autoencoder 90 in which the restoration accuracy reaches the setting level in this way is stored in the storage 30 of the evaluation support apparatus 10 as the extraction model 80. In addition, in a case where the series of processes is repeated a set number of times, the learning may be ended, regardless of the restoration accuracy from the reference patch image 85RL for learning to the restored image 93L for learning.

The learning of the autoencoder 90 may be performed by the evaluation support apparatus 10 or may be performed by a device different from the evaluation support apparatus 10. In the latter case, the extraction model 80 is transmitted from another device to the evaluation support apparatus 10, and the RW control unit 50 stores the extraction model 80 in the storage 30.

As shown in FIG. 13 as an example, the reference patch image 85RL for learning is supplied from a plurality of reference patch images 85R obtained by subdividing a reference region image 70R. The reference region image 70R is an image in which a tissue specimen of the subject S of a past control group 26P is imaged. The past control group 26P is composed of a plurality of subjects S to which the candidate substance 27 was not administered in the past evaluation test. Therefore, the number of subjects S constituting the past control group 26P is significantly larger than the number of subjects S constituting the dose group 25 and the control group 26, and is, for example, about several hundred to several thousand. Since the reference region image 70R is also obtained in a plurality from one subject S as in the region image 70, the number of reference region images 70R obtained from the past control group 26P is obtained by multiplying the number of reference region images 70R obtained from one subject S by the number of subjects S. The tissue specimen of the subject S of the past control group 26P is an example of a "tissue specimen considered to be normal" according to the technology of the present disclosure. It should be noted that, not only the region image 70 in which the tissue specimen of the subject S of the past control group 26P is imaged but also a region image 70 in which a tissue specimen determined to be normal by a specialist such as a pathologist is imaged in a past dose group composed of a plurality of subjects S to which the candidate substance is administered in the past evaluation test may be adopted as the reference region image 70R.

FIG. 13 illustrates the reference region image 70R in which the liver specimen LVS is imaged. The encoder unit 91 of the autoencoder 90 trained by using the reference patch image 85RL for learning based on the reference region image 70R in which the liver specimen LVS is imaged is used as the liver specimen extraction model 80C. Similarly, for example, the encoder unit 91 of the autoencoder 90 trained by using the reference patch image 85RL for learning based on the reference region image 70R in which the brain specimen BS is imaged is used as the brain specimen extraction model 80A.

Next, a configuration of the extraction reference information 44 will be described. First, as shown in FIG. 14 as an example, a plurality of reference feature amounts 87R are extracted from each of a plurality of reference patch images 85R based on all of the plurality of reference region images 70R by using the extraction model 80.

A graph 100 shown in FIG. 15 as an example is a graph in which the plurality of reference feature amounts 87R extracted in FIG. 14 are plotted in the feature amount space 101. The extraction reference information 44 includes coordinates 102 (hereinafter, referred to as representative position coordinates) of a representative position of the reference feature amount 87R indicated by an X mark in the feature amount space 101. The representative position is, for example, a center point or an average point of a distribution 103 of the reference feature amount 87R. In addition, the extraction reference information 44 also includes a determination threshold value 104. In addition, in FIG. 15, for convenience of description, the dimensions of the feature amount space 101 are set to two dimensions having a D1-axis and a D2-axis, but the actual dimensions of the feature amount space 101 are, for example, 512 dimensions as described above. Similarly, in FIG. 16 and the like, which will be described below, for convenience of description, the dimension of the feature amount space 101 is represented in two dimensions.

As in the learning of the autoencoder 90, the representative position coordinates 102 of the extraction reference information 44 may be derived by the evaluation support apparatus 10 or may be derived by a device different from the evaluation support apparatus 10. In the latter case, the representative position coordinates 102 are transmitted from another device to the evaluation support apparatus 10, and the RW control unit 50 stores the representative position coordinates 102 in the storage 30.

As shown in FIG. 16 as an example, the extraction unit 53 calculates a distance D between a representative position of the reference feature amount 87R represented by the representative position coordinates 102 of the extraction reference information 44 and a position of the feature amount 87 in the feature amount space 101. The extraction unit 53 calculates the distance D between the plurality of feature amounts 87 extracted for each of the plurality of patch images 85 obtained by subdividing one region image 70. The distance D is a Mahalanobis distance. The distance D indicates a degree of deviation of the feature amount 87 from the reference feature amount 87R, further indicating a degree of deviation of the tissue specimen imaged in the patch image 85 from the tissue specimen considered to be normal. That is, the larger the distance D is, the more the tissue specimen imaged in the patch image 85 deviates from the tissue specimen considered to be normal. Therefore, the larger the distance D is, the higher the possibility that the morphological abnormality has occurred in the tissue specimen imaged in the patch image 85.

As the distance D, any of an average value, a median value, or a maximum value of a Euclidean distance between a position of a k-nearest neighbor sample of the distribution 103 of the reference feature amount 87R and the position of the feature amount 87 may be calculated. Alternatively, instead of the distance D, a value obtained by subtracting a cosine similarity between a vector representing the representative position of the reference feature amount 87R and a vector representing the position of the feature amount 87 from 1.0 may be calculated. The cosine similarity takes a value between -1.0 and 1.0, and it can be said that the larger the value is, the more similar the directions of the vectors are.

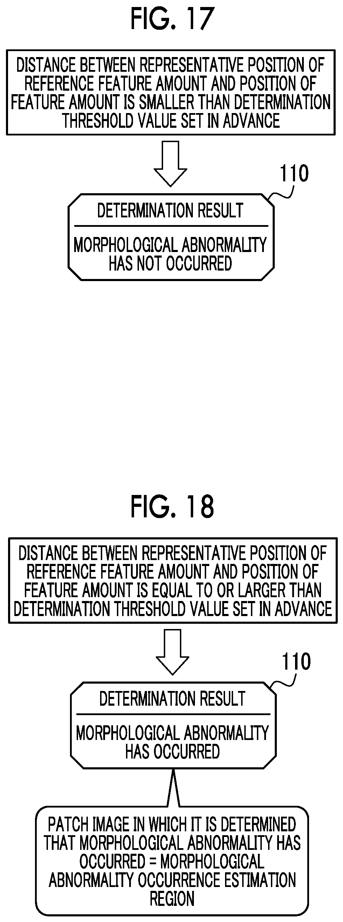

As shown in FIGS. 17 and 18 as an example, the extraction unit 53 compares magnitudes of the calculated distance D and the determination threshold value 104. As shown in FIG. 17, in a case where the distance D is smaller than the determination threshold value 104, the extraction unit 53 determines that the morphological abnormality has not occurred in the tissue specimen imaged in the patch image 85. The extraction unit 53 outputs a determination result 110 indicating that the morphological abnormality has not occurred in the tissue specimen imaged in the patch image 85.

On the other hand, as shown in FIG. 18, in a case where the distance D is equal to or larger than the determination threshold value 104, the extraction unit 53 determines that the morphological abnormality has occurred in the tissue specimen imaged in the patch image 85. The extraction unit 53 outputs the determination result 110 indicating that the morphological abnormality has occurred in the tissue specimen imaged in the patch image 85. The region of the patch image 85 in which it is determined that the morphological abnormality has occurred in the tissue specimen in this way corresponds to the morphological abnormality occurrence estimation region, that is, the "region in which the morphological abnormality is estimated to have occurred" according to the technology of the present disclosure. The extraction result 63 output from the extraction unit 53 is a set of the patch images 85 in which the determination result 110 indicating that the morphological abnormality has occurred in the tissue specimen is output. The determination threshold value 104 may be common to the dose group 25 and the control group 26 or may be different between these groups. In addition, instead of the distance D, it may be determined whether or not the morphological abnormality has occurred in the tissue specimen imaged in the patch image 85 by comparing the cosine similarity and the determination threshold value 104.

As shown in FIGS. 13 and 14, the reference feature amount 87R is a feature amount extracted from the reference patch image 85R obtained by subdividing the reference region image 70R in which the tissue specimen of the subject S of the past control group 26P is imaged. The subject S of the past control group 26P is the subject S to which the candidate substance 27 is not administered. Therefore, at least the morphological abnormality caused by the toxicity of the candidate substance 27 has not occurred in the tissue specimen imaged in the reference region image 70R. Therefore, the representative position of the reference feature amount 87R is regarded as the representative position of the feature amount of the region image 70 in which the normal tissue specimen is imaged. Therefore, as described above, the distance D between the representative position of the reference feature amount 87R and the position of the feature amount 87 is an indicator indicating how much the tissue specimen imaged in the patch image 85 deviates from the normal tissue specimen. Therefore, as shown in FIG. 17, the extraction unit 53 determines that the morphological abnormality has not occurred for the patch image 85 in which the distance D is smaller than the determination threshold value 104, on the assumption that the imaged tissue specimen does not deviate from the normal tissue specimen. On the other hand, as shown in FIG. 18, the extraction unit 53 determines that the morphological abnormality has occurred for the patch image 85 in which the distance D is equal to or larger than the determination threshold value 104, on the assumption that the imaged tissue specimen deviates from the normal tissue specimen.

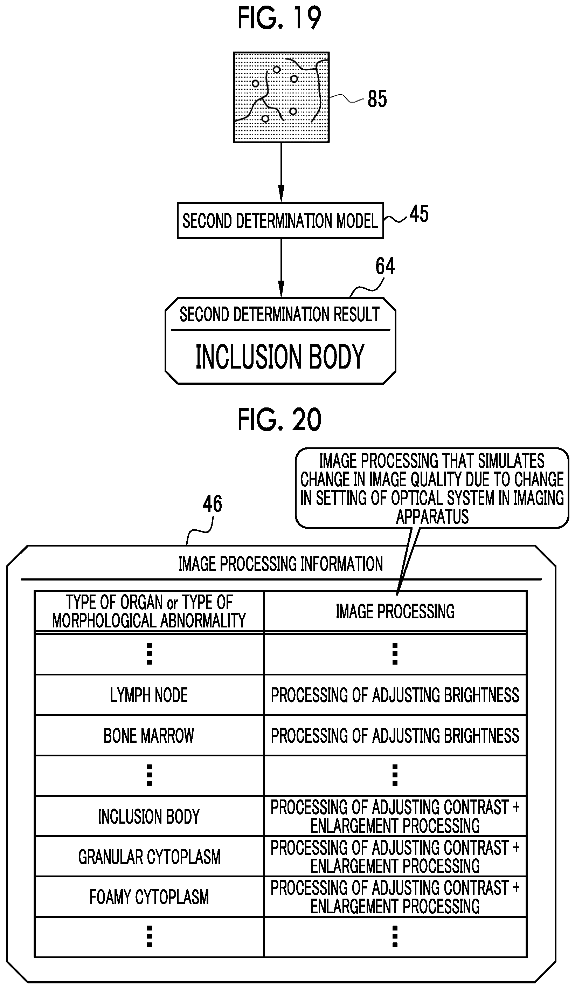

As shown in FIG. 19 as an example, the second determination unit 54 inputs the patch image 85 to the second determination model 45. Then, the second determination model 45 determines the type of the morphological abnormality estimated to have occurred in the tissue specimen imaged in the patch image 85. The second determination model 45 is also, for example, a machine learning model such as a convolutional neural network, as in the identification model 41 and the like. The second determination model 45 outputs the second determination result 64 of the type of the morphological abnormality estimated to have occurred in the tissue specimen imaged in the patch image 85. In FIG. 19, the inclusion body is illustrated as the type of the morphological abnormality.

As shown in FIG. 20 as an example, the image processing information 46 is information in which the image processing performed on the region image 70 or the patch image 85 by the image processing unit 55 is summarized. In the image processing information 46, the image processing suitable for the organ or the morphological abnormality is registered for each type of organ or each type of morphological abnormality. The image processing is image processing that simulates a change in image quality due to a change in setting of an optical system in the imaging apparatus 19. Specifically, the image processing includes processing of adjusting brightness in a case where the type of the organ is a lymph node or a bone marrow. In addition, the image processing includes processing of adjusting contrast and enlargement processing in a case where the type of the morphological abnormality is an inclusion body, granular cytoplasm, or foamy cytoplasm.

The processing of adjusting brightness is processing that simulates a change in which the brightness of the image increases in a case where an aperture stop of the imaging apparatus 19 is opened. Therefore, the processing of adjusting brightness may be rephrased as processing of increasing brightness. The processing of adjusting contrast is processing that simulates a change in which the contrast of brightness and darkness and/or the contrast of color of the image increases in a case where the aperture stop and a field stop of the imaging apparatus 19 are narrowed. Therefore, the processing of adjusting contrast may be rephrased as processing of increasing contrast. The enlargement processing is processing that simulates a change in which the image is enlarged in a case where the magnification of the objective lens of the imaging apparatus 19 is increased, and is so-called electronic zoom processing. Brightness adjusted by the processing of adjusting brightness and contrast adjusted by the processing of adjusting contrast are set to a defined level in advance. In addition, a magnification enlarged by the enlargement processing is also set to a defined magnification (for example, 1.25 times to 1.5 times) in advance. The aperture stop, the field stop, and the objective lens are examples of an "optical system" according to the technology of the present disclosure.

In a case where the type of the organ is the lymph node or the bone marrow, it is recommended to increase the brightness in the imaging apparatus 19 to observe the organ. Therefore, the processing of adjusting brightness is registered as described above. The inclusion body, the granular cytoplasm, and the foamy cytoplasm are extremely small and discrete because they occur in cells. Therefore, in a case where the type of the morphological abnormality is the inclusion body, the granular cytoplasm, or the foamy cytoplasm, the processing of adjusting contrast and the enlargement processing are registered as described above.

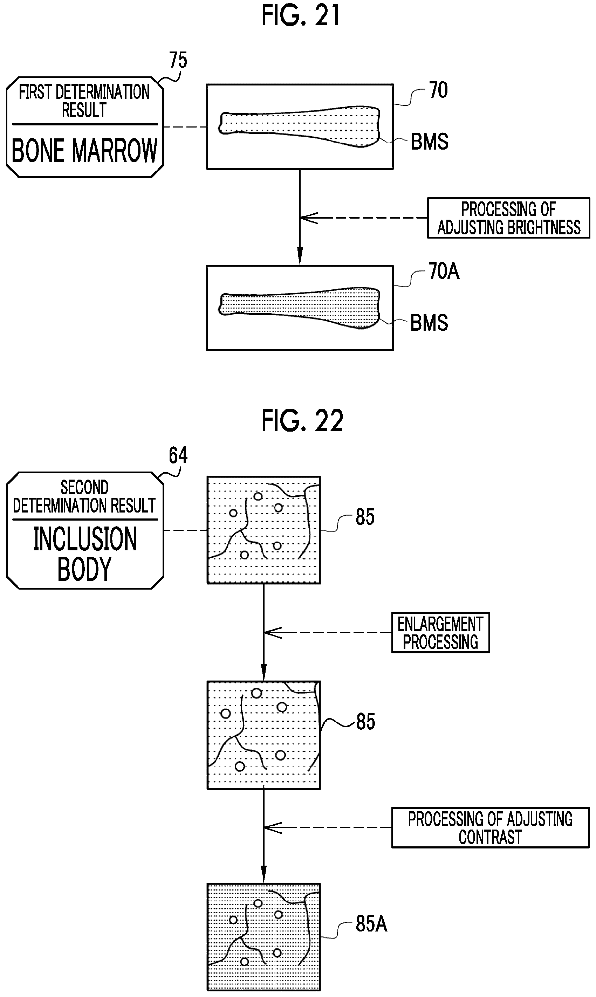

As shown in FIG. 21 as an example, in a case where the type of the organ of the first determination result 75 is the type of the organ for which the image processing suitable for the image processing information 46 is registered, the image processing unit 55 performs the image processing registered in the image processing information 46 on the region image 70. Hereinafter, the region image 70 after the image processing will be referred to as a post-processing region image 70A. FIG. 21 illustrates a case where the tissue specimen imaged in the region image 70 is the bone marrow specimen BMS, the type of the organ of the first determination result 75 is the bone marrow, and the processing of adjusting brightness is performed on the region image 70.

In addition, as shown in FIG. 22 as an example, in a case where the type of the morphological abnormality of the second determination result 64 is the type of the morphological abnormality for which the image processing suitable for the image processing information 46 is registered, the image processing unit 55 performs the image processing registered in the image processing information 46 on the patch image 85. Hereinafter, the patch image 85 after the image processing will be referred to as a post-processing patch image 85A. FIG. 22 illustrates a case where the type of the morphological abnormality of the second determination result 64 is the inclusion body, and the processing of adjusting contrast and the enlargement processing (the order of performing is the enlargement processing and the processing of adjusting contrast) are performed on the patch image 85.

The display control unit 56 performs control of displaying, on the display 11, the image list display screen 120 shown in FIG. 23 as an example in response to the display instruction from the user U through the input device 12. The image list display screen 120 includes a display region 121. All the region images 70 generated by the identification unit 51 are displayed in a list in the display region 121. In the display region 121, the region images 70 are arranged in order of the region image ID from top to bottom and from left to right. An organ name based on the first determination result 75 is displayed in the region image 70 together with the region image ID. An OK button 122 is provided below the display region 121. In a case where the OK button 122 is selected, the display control unit 56 erases the display of the image list display screen 120. The target of the region image 70 to be displayed on the image list display screen 120 may be limited to only one subject S or only the high-dose group 25H.

Each region image 70 in the display region 121 can be selected. In a case where the region image 70 is selected by the user U and an enlarged display instruction of the region image 70 is issued, the display control unit 56 performs control of displaying the first image display screen 125 shown in FIG. 24 as an example on the display 11. The first image display screen 125 is a screen on which the region image 70 selected by the user U on the image list display screen 120 is enlarged and displayed. In a case where the image processing is performed on the region image 70 by the image processing unit 55, the post-processing region image 70A is enlarged and displayed on the first image display screen 125, and a message indicating that the image processing is performed on the region image 70 is displayed. In addition, in a case where the image processing is performed on the region image 70 by the image processing unit 55, a return button 126 for returning the post-processing region image 70A to the region image 70 before the image processing is performed is provided on the first image display screen 125. In a case where the image processing is not performed on the region image 70 by the image processing unit 55, the region image 70 is enlarged and displayed on the first image display screen 125, and the message and the return button 126 are not displayed. FIG. 24 illustrates a case where the tissue specimen imaged in the region image 70 is the bone marrow specimen BMS, and the processing of adjusting brightness is performed on the region image 70 as the image processing.

An analysis button 127 and an OK button 128 are provided at a lower part of the first image display screen 125. In a case where the analysis button 127 is selected and an analysis instruction of the region image 70 is issued, the extraction unit 53 extracts the morphological abnormality occurrence estimation region from the region image 70 or the post-processing region image 70A displayed on the first image display screen 125. On the other hand, in a case where the OK button 128 is selected, the display control unit 56 erases the display of the first image display screen 125.

In a case where the extraction result 63 from the extraction unit 53 is input, as shown in FIG. 25 as an example, the display control unit 56 displays a frame 130 indicating the patch image 85 extracted as the morphological abnormality occurrence estimation region in a superimposed manner on the tissue specimen of the region image 70 or the post-processing region image 70A. FIG. 25 illustrates a case where the tissue specimen imaged in the region image 70 is the liver specimen LVS.

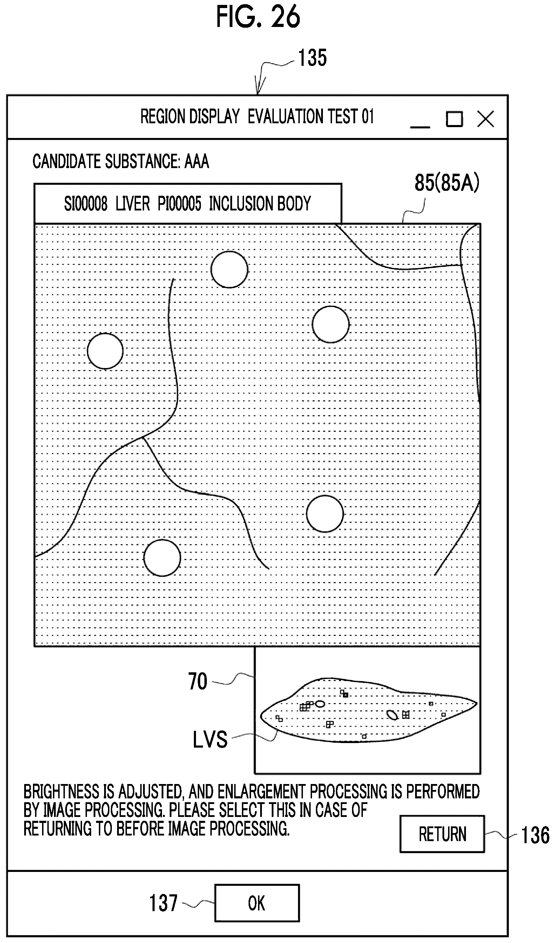

The frame 130 can be selected. In a case where the frame 130 is selected by the user U and an enlarged display instruction of the patch image 85 indicated by the frame 130 is issued, the display control unit 56 performs control of displaying a second image display screen 135 shown in FIG. 26 on the display 11. The second image display screen 135 is a screen on which the patch image 85 indicated by the frame 130 selected by the user U is enlarged and displayed. In a case where the image processing is performed on the patch image 85 by the image processing unit 55, the post-processing patch image 85A is enlarged and displayed on the second image display screen 135, and a message indicating that the image processing is performed on the patch image 85 is displayed. In addition, in a case where the image processing is performed on the patch image 85 by the image processing unit 55, a return button 136 for returning the post-processing patch image 85A to the patch image 85 before the image processing is performed is provided on the second image display screen 135. In a case where the image processing is not performed on the patch image 85 by the image processing unit 55, the patch image 85 is enlarged and displayed on the second image display screen 135, and the message and the return button 136 are not displayed. FIG. 26 illustrates a case where the type of the morphological abnormality estimated to have occurred in the tissue specimen imaged in the patch image 85 is the inclusion body, and the processing of adjusting contrast and the enlargement processing are performed on the patch image 85 as the image processing.

The region image 70 in which the frame 130 is displayed in a superimposed manner on the tissue specimen is reduced and displayed at a lower part of the patch image 85 or 85A. In the reduced region image 70, the display control unit 56 displays the frame 130 corresponding to the currently displayed patch image 85 or 85A in a distinguishable manner from other frames 130 by changing a color of the frame 130 or the like. In addition, an OK button 137 is provided at a lower part of the second image display screen 135. In a case where the OK button 137 is selected, the display control unit 56 erases the display of the second image display screen 135.

In a case where the region image 70 enlarged and displayed on the first image display screen 125 is obtained from the subject S belonging to the dose group 25, the region image 70 of the same organ obtained from the subject S of the control group 26 may be displayed side by side for comparison. Alternatively, in the image list display screen 120, the region image 70 obtained from the subject S belonging to the dose group 25 and the region image 70 obtained from the subject S belonging to the control group 26 may be displayed side by side.

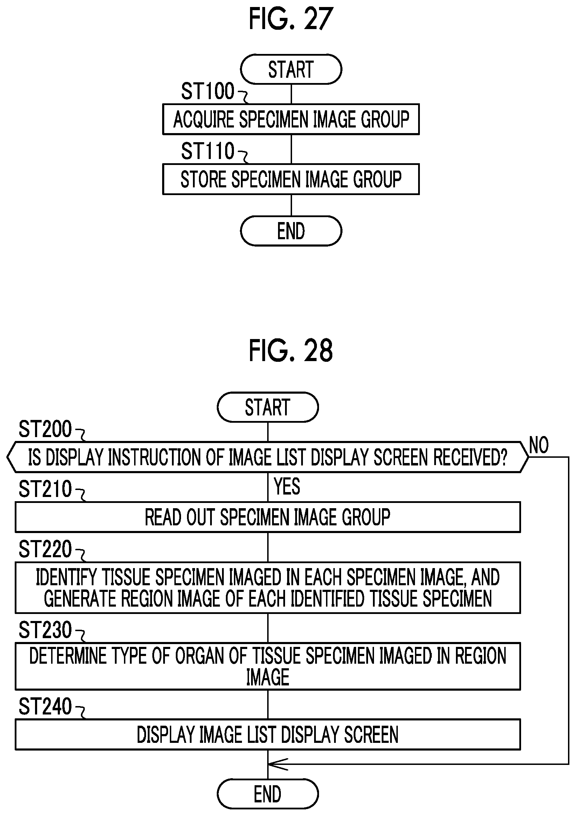

Next, an operation of the configuration described above will be described with reference to the flowchart shown in FIGS. 27 to 31 as an example. First, in a case where the operation program 40 is activated in the evaluation support apparatus 10, as shown in FIG. 4, the CPU 32 of the evaluation support apparatus 10 functions as the RW control unit 50, the identification unit 51, the first determination unit 52, the extraction unit 53, the second determination unit 54, the image processing unit 55, and the display control unit 56.

The imaging apparatus 19 captures the specimen image 15 of the tissue specimen of the subject S. The specimen image 15 is transmitted from the imaging apparatus 19 to the evaluation support apparatus 10. In the evaluation support apparatus 10, as shown in FIG. 27, the specimen image group 60, which is a set of the specimen images 15 from the imaging apparatus 19, is acquired by the RW control unit 50 (step ST100). The specimen image group 60 is stored in the storage 30 under the control of the RW control unit 50 (step ST110).

In FIG. 28, in a case where the display instruction of the image list display screen 120 is issued by the user U through the input device 12 and the display instruction is received by the CPU 32 (YES in step ST200), the specimen image group 60 designated by the display instruction is read out from the storage 30 by the RW control unit 50 (step ST210). The specimen image group 60 is output from the RW control unit 50 to the identification unit 51 and the display control unit 56.

In the identification unit 51, as shown in FIG. 5, the specimen image 15 is input to the identification model 41, and the plurality of tissue specimens imaged in the specimen image 15 are identified, and the identification result is output from the identification model 41. Then, the region image 70 of each tissue specimen is generated based on the identification result (step ST220). The region image group 61, which is a set of the region images 70, is output from the identification unit 51 to the first determination unit 52.

In the first determination unit 52, as shown in FIGS. 6 and 7, the region image 70 is input to the first determination model 42. As a result, the type of the organ of the tissue specimen imaged in the region image 70 is determined (step ST230), and the first determination result 75 is output from the first determination model 42. The first determination result group 62, which is a set of the first determination results 75, is output to the image processing unit 55 and the display control unit 56. As shown in FIG. 23, the image list display screen 120 in which the region images 70 are arranged in the display region 121 is displayed on the display 11 under the control of the display control unit 56(step ST240).

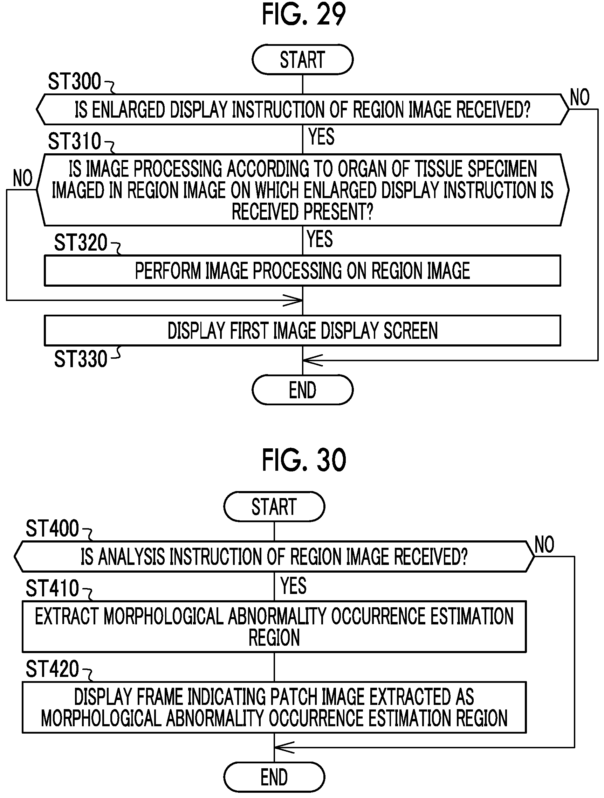

In FIG. 29, in a case where one of the region images 70 displayed in a list on the image list display screen 120 is selected by the user U and the enlarged display instruction of the region image 70 is received by the CPU 32 (YES in step ST300), the image processing information 46 and the first determination result 75 are referred to in the image processing unit 55. Then, it is searched whether or not the image processing according to the type of the organ of the tissue specimen imaged in the region image 70 on which the enlarged display instruction is received is registered in the image processing information 46 (step ST310).

In a case where the image processing according to the organ of the tissue specimen imaged in the region image 70 on which the enlarged display instruction is received is registered in the image processing information 46 (YES in step ST310), as shown in FIG. 21, the image processing unit 55 performs the image processing registered in the image processing information 46 on the region image 70 (step ST320). On the other hand, in a case where the image processing according to the organ of the tissue specimen imaged in the region image 70 on which the enlarged display instruction is received is not registered in the image processing information 46 (NO in step ST310), the image processing is not performed on the region image 70. As shown in FIG. 24, the first image display screen 125 on which the region image 70 or the post-processing region image 70A is enlarged and displayed is displayed on the display 11 under the control of the display control unit 56 (step ST330).

In FIG. 30, in a case where the analysis button 127 is selected by the user U and the analysis instruction of the region image 70 is received by the CPU 32 (YES in step ST400), as shown in FIGS. 9, 10, and 16 to 18, the extraction unit 53 extracts the morphological abnormality occurrence estimation region (step ST410).

Specifically, first, as shown in FIG. 9, the region image 70 is subdivided into the patch image 85. Next, as shown in FIG. 10, the patch image 85 is input to the extraction model 80, and the feature amount 87 is output from the extraction model 80. Then, as shown in FIG. 16, the distance D between the feature amount 87 and the representative position of the reference feature amount 87R in the feature amount space 101 is calculated. Finally, as shown in FIGS. 17 and 18, the magnitudes of the distance D and the determination threshold value 104 are compared.

In a case where the distance D is smaller than the determination threshold value 104, the determination result 110 indicating that the morphological abnormality has not occurred in the tissue specimen imaged in the patch image 85 is output. On the other hand, in a case where the distance D is equal to or larger than the determination threshold value 104, the determination result 110 indicating that the morphological abnormality has occurred in the tissue specimen imaged in the patch image 85 is output. A set of the patch images 85 in which the determination result 110 indicating that the morphological abnormality has occurred is output is output from the extraction unit 53 to the second determination unit 54 and the display control unit 56 as the extraction result 63 of the morphological abnormality occurrence estimation region. As shown in FIG. 25, the frame 130 indicating the patch image 85 extracted as the morphological abnormality occurrence estimation region is displayed in a superimposed manner on the tissue specimen of the region image 70 or the post-processing region image 70A under the control of the display control unit 56 (step ST420).

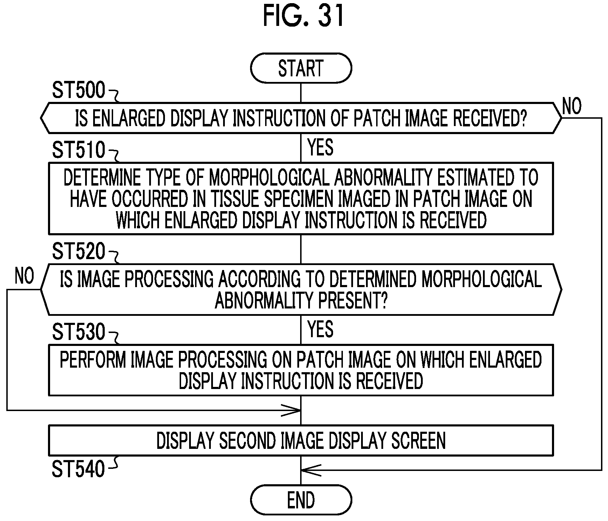

In FIG. 31, in a case where the frame 130 is selected by the user U and the enlarged display instruction of the patch image 85 indicated by the frame 130 is received by the CPU 32 (YES in step ST500), the second determination unit 54 determines the type of the morphological abnormality estimated to have occurred in the tissue specimen imaged in the patch image 85 on which the enlarged display instruction is received (step ST510). Specifically, as shown in FIG. 19, the patch image 85 on which the enlarged display instruction is received is input to the second determination model 45, and the second determination result 64 of the type of the morphological abnormality is output from the second determination model 45. The second determination result 64 is output from the second determination unit 54 to the image processing unit 55 and the display control unit 56.

In the image processing unit 55, the image processing information 46 and the second determination result 64 are referred to, and it is searched whether or not the image processing according to the type of the morphological abnormality of the second determination result 64 is registered in the image processing information 46 (step ST520). In a case where the image processing according to the type of the morphological abnormality of the second determination result 64 is registered in the image processing information 46 (YES in step ST520), as shown in FIG. 22, the image processing unit 55 performs the image processing registered in the image processing information 46 on the patch image 85 (step ST530). On the other hand, in a case where the image processing according to the type of the morphological abnormality of the second determination result 64 is not registered in the image processing information 46 (NO in step ST520), the image processing is not performed on the patch image 85. As shown in FIG. 26, the second image display screen 135 on which the patch image 85 or the post-processing patch image 85A is enlarged and displayed is displayed on the display 11 under the control of the display control unit 56 (step ST540).

The user U evaluates the candidate substance 27 by observing the region image 70 or the post-processing region image 70A in detail on the first image display screen 125 or by observing the patch image 85 or the post-processing patch image 85A in detail on the second image display screen 135.

As described above, the CPU 32 of the evaluation support apparatus 10 comprises the RW control unit 50 and the image processing unit 55. The RW control unit 50 acquires the specimen image 15 in which the tissue specimen of the subject S provided for the evaluation test of the candidate substance 27 of the drug is imaged. The image processing unit 55 performs the image processing according to the type of the source organ of the tissue specimen and/or the type of the morphological abnormality estimated to have occurred in the tissue specimen on the region image 70 or the patch image 85. Therefore, the candidate substance 27 can be evaluated by the post-processing region image 70A on which the image processing suitable for the type of the organ is performed or the post-processing patch image 85A on which the image processing suitable for the type of the morphological abnormality is performed. Therefore, it is possible to suppress a decrease in accuracy of the evaluation of the candidate substance 27.

As shown in FIG. 20, the image processing is image processing that simulates a change in image quality of the specimen image 15 due to a change in setting of the optical system in the imaging apparatus 19. Therefore, in a case where the specimen image 15 is captured by the imaging apparatus 19, the optical system need not be strictly changed in setting each time according to the type of the organ or the type of the morphological abnormality. It is possible to significantly reduce the burden on the user U in a case where the specimen image 15 is captured.

The image processing includes at least one of processing of adjusting brightness, processing of adjusting contrast, or enlargement processing. Therefore, the candidate substance 27 can be evaluated by the post-processing region image 70A or the post-processing patch image 85A in an appropriate state of brightness, contrast, or magnification. It is possible to further suppress a decrease in accuracy of the evaluation of the candidate substance 27.

As shown in FIG. 22, the image processing unit 55 performs the image processing only on the patch image 85 extracted as the morphological abnormality occurrence estimation region. Therefore, it is possible to reduce a load and time required for the image processing as compared with a case where the image processing is performed on the entire region image 70.

The first determination unit 52 determines the type of the source organ of the tissue specimen. In addition, the second determination unit 54 determines the type of the morphological abnormality. Therefore, it is possible to significantly reduce the burden on the user U as compared with a case where the user U visually determines the type of the source organ of the tissue specimen and/or the type of the morphological abnormality and inputs the determination result to the evaluation support apparatus 10 through the input device 12.

The extraction unit 53 extracts the morphological abnormality occurrence estimation region from the region image 70. Therefore, it is possible to significantly reduce the burden on the user U as compared with a case where the user U visually extracts the morphological abnormality occurrence estimation region.

The extraction unit 53 extracts the morphological abnormality occurrence estimation region by using the extraction model 80 that is the machine learning model. In recent years, the machine learning model has made remarkable progress, and it is possible to easily prepare a relatively high-accuracy machine learning model. Therefore, it is possible to easily and accurately extract the morphological abnormality occurrence estimation region.

The extraction unit 53 extracts the morphological abnormality occurrence estimation region by comparing the feature amount 87 obtained by inputting the patch image 85 to the extraction model 80 with the reference feature amount 87R obtained by inputting the reference patch image 85R of the reference region image 70R in which the tissue specimen considered to be normal is imaged to the extraction model 80. Therefore, it is possible to more easily and accurately extract the morphological abnormality occurrence estimation region.

### [Second Embodiment]

In the first embodiment, the image processing unit 55 automatically performs the image processing, but the present disclosure is not limited to this. In a case where the instruction to perform the image processing from the user U is received by the CPU 32, the image processing unit 55 may perform the image processing.

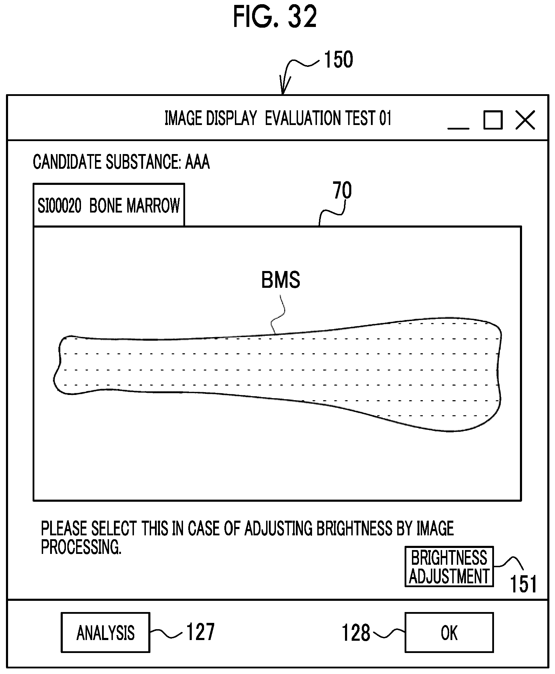

As shown in FIG. 32 as an example, in a case where the type of the organ of the first determination result 75 is the type of the organ for which the image processing suitable for the image processing information 46 is registered, a button for issuing an instruction to perform the image processing is provided on a first image display screen 150 of the second embodiment. FIG. 32 illustrates a case where the tissue specimen imaged in the region image 70 is the bone marrow specimen BMS. In this case, a brightness adjustment button 151 is provided on the first image display screen 150 as the button for issuing the instruction to perform the image processing. In a case where the brightness adjustment button 151 is selected by the user U, the CPU 32 receives an instruction to perform the processing of adjusting the brightness. In response to this, the image processing unit 55 performs the processing of adjusting the brightness on the region image 70.

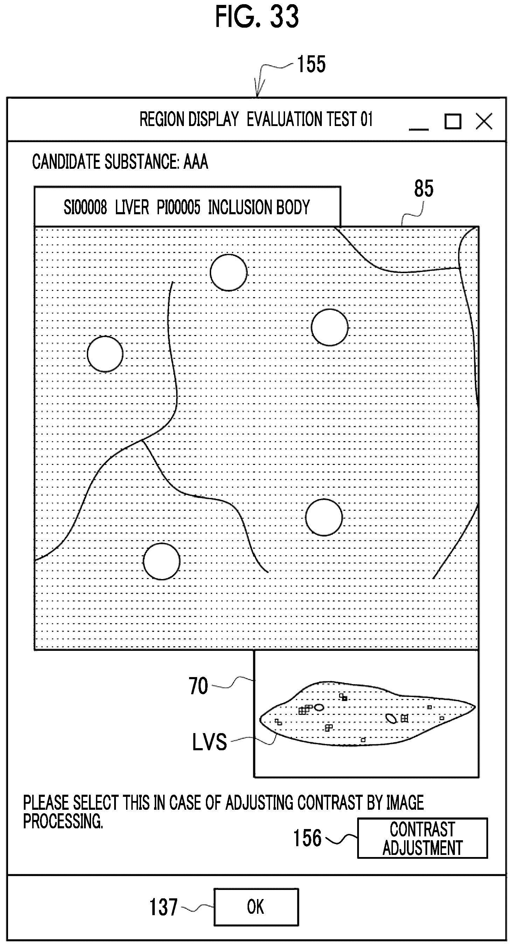

In addition, as shown in FIG. 33 as an example, in a case where the type of the morphological abnormality of the second determination result 64 is the type of the morphological abnormality for which the image processing suitable for the image processing information 46 is registered, a button for issuing an instruction to perform the image processing is provided on a second image display screen 155 of the second embodiment. FIG. 33 illustrates a case where the type of the morphological abnormality estimated to have occurred in the tissue specimen imaged in the patch image 85 is the inclusion body. In this case, a contrast adjustment button 156 is provided on the second image display screen 155 as the button for issuing the instruction to perform the image processing. In a case where the contrast adjustment button 156 is selected by the user U, the CPU 32 receives an instruction to perform the processing of adjusting the contrast. In response to this, the image processing unit 55 performs the processing of adjusting the contrast on the patch image 85. The image processing unit 55 automatically performs the enlargement processing on the patch image 85.

As described above, in the second embodiment, the instruction to perform the image processing from the user U is received. Therefore, it is possible to decide whether or not to perform the image processing by the determination of the user U. In a case where the user U actually observes the region image 70 or the patch image 85 and determines that the image processing is not necessary, unnecessary image processing need not be performed.

A plurality of buttons for issuing an instruction to perform various types of image processing, for example, a brightness adjustment button, a contrast adjustment button, and an enlarged display button may be provided on the first image display screen and the second image display screen, the user U may be caused to determine the type of the organ or the type of the morphological abnormality, and the user U may be caused to issue the instruction to perform the image processing. For example, in a case where the tissue specimen imaged in the region image 70 is the bone marrow specimen BMS, the user U observes the region image 70 and determines that the type of the organ is the bone marrow. Then, the brightness adjustment button among the plurality of buttons is selected to cause the image processing unit 55 to perform the processing of adjusting the brightness.

### [Third Embodiment]

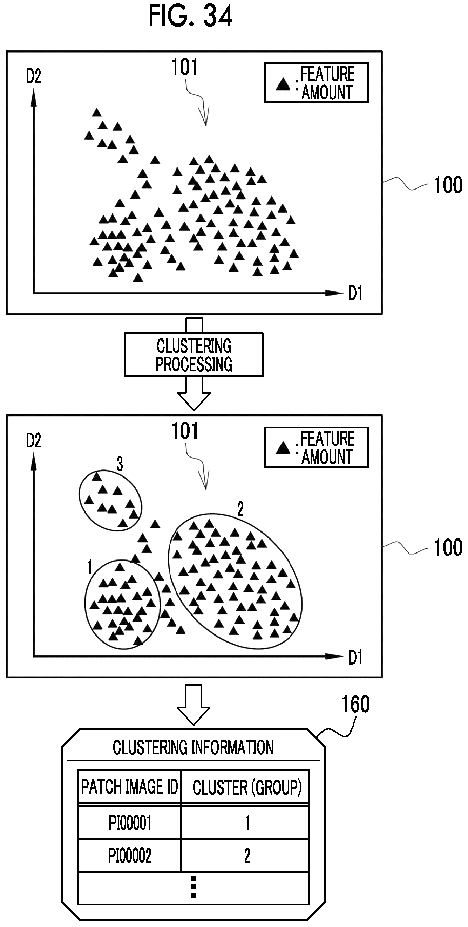

As shown in FIG. 34 as an example, in the third embodiment, the extraction unit 53 performs clustering processing of defining a cluster to which each of the patch images 85 extracted as the morphological abnormality occurrence estimation region belongs, on the feature amount 87 of the patch image 85. As the clustering processing, a k-means method, a hierarchical density-based spatial clustering (HDBSCAN), a Gaussian mixture model (GMM), a probabilistic latent semantic analysis (PLSA), a non-negative matrix factorization (NMF), a fuzzy c-means (FCM) method, or the like can be used. FIG. 34 shows an example in which the feature amount 87 is clustered into three clusters of a cluster 1, a cluster 2, and a cluster 3. As illustrated, some of the feature amounts 87 do not belong to any of the clusters. The clusters 1 to 3 are examples of a "group" according to the technology of the present disclosure.

The extraction unit 53 generates clustering information 160. The clustering information 160 is information in which the cluster to which the patch image 85 belongs is registered for each patch image ID of the patch image 85. The patch image 85 of which the feature amount 87 does not belong to any of the clusters is not registered in the clustering information 160. The extraction unit 53 outputs the clustering information 160 to the display control unit 56.

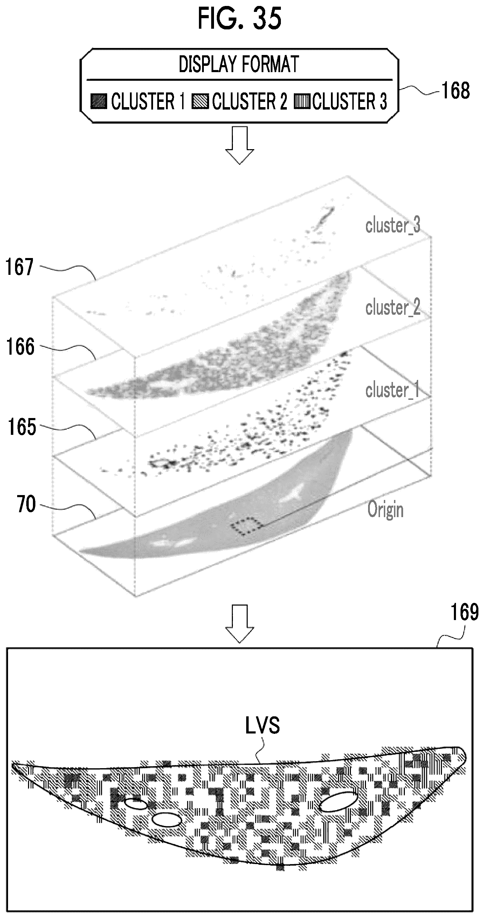

As shown in FIG. 35 as an example, the display control unit 56 generates cluster images 165, 166, and 167 by processing the region image 70 according to the clustering information 160. The cluster image 165 is an image corresponding to the cluster 1. The cluster image 166 is an image corresponding to the cluster 2. The cluster image 167 is an image corresponding to the cluster 3.

The display control unit 56 generates the cluster images 165 to 167 according to a display format 168 set in advance for each cluster. The display format 168 is, for example, a content in which the cluster 1 is displayed in indigo, the cluster 2 is displayed in yellow-green, and the cluster 3 is displayed in gray. The display control unit 56 generates the cluster image 165 by filling a position (which can be found from position information) of the patch image 85 of the patch image ID registered in the clustering information 160 in the cluster 1 in the region image 70 with indigo. Similarly, the display control unit 56 generates the cluster image 166 by filling a position of the patch image 85 of the patch image ID registered in the clustering information 160 in the cluster 2 in the region image 70 with yellow-green. Further, the display control unit 56 generates the cluster image 167 by filling a position of the patch image 85 of the patch image ID registered in the clustering information 160 in the cluster 3 in the region image 70 with gray. By changing the color displayed in this way, the cluster images 165 to 167 are images in which the clusters 1 to 3 can be identified. The display format 168 may be configured to be freely changed in setting by the user U.

The display control unit 56 generates a superimposition image 169 in which the region image 70 and at least one of the cluster images 165 to 167 are superimposed. FIG. 35 illustrates the superimposition image 169 in which all of the cluster images 165 to 167 are superimposed on the region image 70.

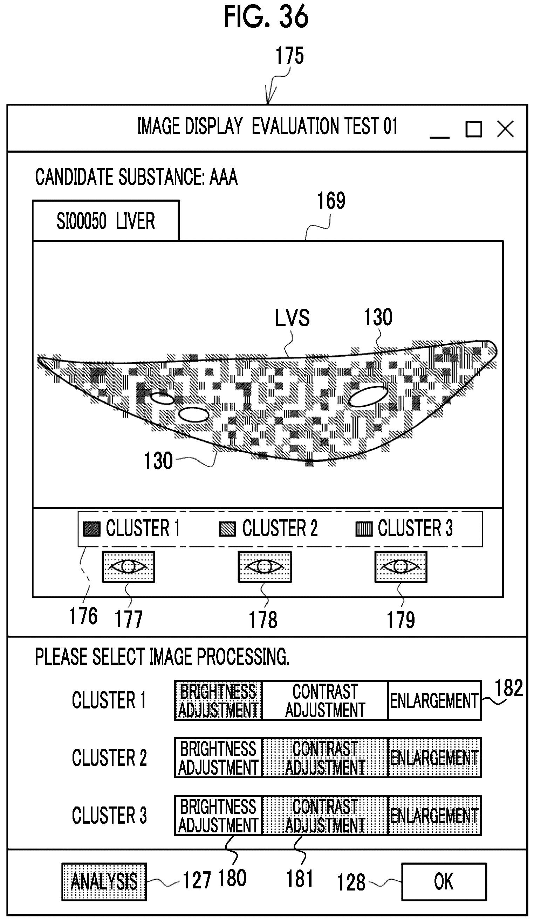

In a case where the analysis button 127 is selected and the analysis instruction of the region image 70 is issued and the extraction unit 53 extracts the morphological abnormality occurrence estimation region, the display control unit 56 performs control of displaying a first image display screen 175 shown in FIG. 36 as an example on the display 11. The superimposition image 169 and a legend 176 are displayed on the first image display screen 175. Display switching buttons 177, 178, and 179 are provided at a lower part of the legend 176. The display switching button 177 is a button for selecting whether or not to display the cluster image 165 in a superimposed manner on the region image 70. The display switching button 178 is a button for selecting whether or not to display the cluster image 166 in a superimposed manner on the region image 70. The display switching button 179 is a button for selecting whether or not to display the cluster image 167 in a superimposed manner on the region image 70. Therefore, for example, in a case where all of the display switching buttons 177 to 179 are selected as shown in the drawing, the display control unit 56 displays the superimposition image 169 in which all of the cluster images 165 to 167 are superimposed on the region image 70. As described above, the display control unit 56 displays at least one of the plurality of cluster images 165 to 167 in a superimposed manner on the region image 70. The first image display screen 175 is displayed in a state in which all of the display switching buttons 177 to 179 are selected at first.

A button for issuing an instruction to perform the image processing is provided for each cluster on the first image display screen 175. Specifically, the button for issuing the instruction to perform the image processing is a brightness adjustment button 180, a contrast adjustment button 181, and an enlarged display button 182. The user U determines the type of the morphological abnormality estimated to have occurred in the tissue specimen imaged in the patch image 85 belonging to each cluster, and selects a button corresponding to the determined type of the morphological abnormality from among the buttons 180 to 182.

In a case where the brightness adjustment button 180 is selected by the user U, the CPU 32 receives an instruction to perform the processing of adjusting the brightness. In response to this, the image processing unit 55 performs the processing of adjusting the brightness on all of the patch images 85 belonging to the cluster in a batch. Similarly, in a case where the contrast adjustment button 181 is selected by the user U, the CPU 32 receives an instruction to perform the processing of adjusting the contrast. In response to this, the image processing unit 55 performs the processing of adjusting the contrast on all of the patch images 85 belonging to the cluster in a batch. In addition, in a case where the enlarged display button 182 is selected by the user U, the CPU 32 receives an instruction to perform the enlargement processing. In response to this, the image processing unit 55 performs the enlargement processing on all of the patch images 85 belonging to the cluster in a batch.

As described above, in the third embodiment, the extraction unit 53 classifies the morphological abnormality occurrence estimation region into a plurality of groups (clusters) based on the similarity of the morphological abnormality. The image processing unit 55 performs the same image processing on a group basis. Therefore, it is possible to significantly reduce the burden on the user U in a case where the instruction to perform the image processing is issued as compared with the second embodiment in which the instruction to perform the image processing is required for each patch image 85 one by one.

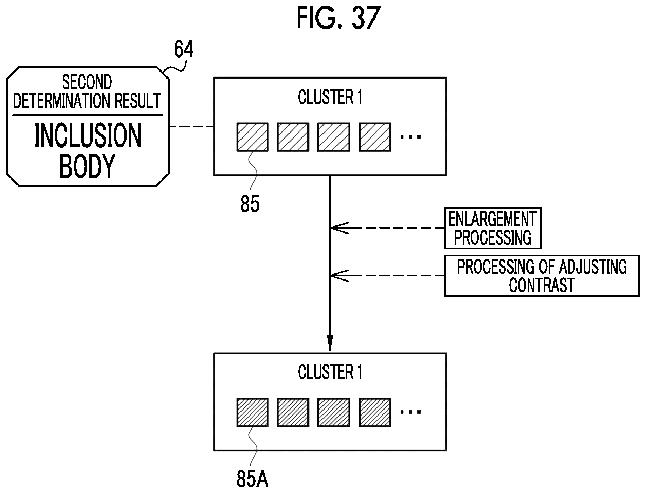

The first embodiment and the third embodiment may be implemented in combination. That is, as shown in FIG. 37 as an example, the second determination unit 54 determines the type of the morphological abnormality estimated to have occurred in the tissue specimen imaged in the patch image 85 belonging to each cluster, instead of the user U. In this case, for example, the type of the morphological abnormality is determined for all of the patch images 85 belonging to the cluster. Then, a plurality of the determined types of the morphological abnormality are determined by a majority vote, and the type of the morphological abnormality that is most frequently determined is determined as the final type of the morphological abnormality. The image processing unit 55 performs the image processing according to the finally determined type of the morphological abnormality on all of the patch images 85 belonging to the cluster in a batch. FIG. 37 illustrates a case where the type of the morphological abnormality estimated to have occurred in the tissue specimen imaged in the patch image 85 belonging to the cluster 1 is determined to be the inclusion body, and the processing of adjusting the contrast and the enlargement processing are performed as the image processing. In this way, it is possible to further reduce the burden on the user U.

Dimensionality reduction processing may be performed on the feature amount 87 before the clustering processing. The dimensionality reduction processing is, for example, processing of converting the 512-dimensional feature amount 87 into the two-dimensional feature amount 87. As the dimensionality reduction processing, principal component analysis (PCA), t-distributed stochastic neighbor embedding (t-SNE), uniform manifold approximation and projection (UMAP), or the like can be used.

The graph 100 of the feature amount 87 shown in FIG. 34 may be displayed on the display 11, and the user U may manually divide the clusters.

The image processing that simulates the change in image quality of the specimen image 15 due to the change in setting of the optical system in the imaging apparatus 19 is not limited to the processing of adjusting the brightness, the processing of adjusting the contrast, and the enlargement processing illustrated as an example. The image processing may be processing of adjusting a resolution of the region image 70 or the patch image 85. The processing of adjusting the resolution is specifically processing of increasing the resolution, and is realized, for example, by performing a known super-resolution technique on the region image 70 or the patch image 85.

In a case where the type of the organ of the first determination result 75 is the type of the organ for which the image processing suitable for the image processing information 46 is registered, the image processing registered in the image processing information 46 is performed on the region image 70, but the present disclosure is not limited to this. The image processing suitable for the type of the organ of the first determination result 75, which is registered in the image processing information 46, may be performed only on the patch image 85 extracted as the morphological abnormality occurrence estimation region, instead of the region image 70.

The determination of the type of the source organ of the tissue specimen imaged in the region image 70 is not limited to the method using the first determination model 42. The type of the organ may be determined by the method described below. That is, a representative feature amount of each organ is acquired in advance by using the machine learning model. In addition, the representative feature amount of the region image 70 that is a target of the determination of the type of the organ is derived by using the machine learning model that derives the representative feature amount of each organ. Next, a distance between the representative feature amount of each organ and the representative feature amount of the region image 70 in the feature amount space is calculated. Then, the organ of the representative feature amount having the shortest distance is determined as the source organ of the tissue specimen imaged in the region image 70.

Similarly, the determination of the type of the morphological abnormality estimated to have occurred in the tissue specimen imaged in the region image 70 is not limited to the method using the second determination model 45. The type of the morphological abnormality may be determined by the method described below. That is, a representative feature amount of each morphological abnormality is acquired in advance by using the machine learning model. In addition, the representative feature amount of the patch image 85 that is a target of the determination of the type of the morphological abnormality is derived by using the machine learning model that derives the representative feature amount of each morphological abnormality. Next, a distance between the representative feature amount of each morphological abnormality and the representative feature amount of the patch image 85 in the feature amount space is calculated. Then, the morphological abnormality of the representative feature amount having the shortest distance is determined as the morphological abnormality estimated to have occurred in the tissue specimen imaged in the patch image 85.

In addition to the reference patch image 85R in which the tissue specimen considered to be normal is imaged, the patch image 85 in which the tissue specimen in which the morphological abnormality has occurred is imaged may be used as the reference patch image 85RL for learning. The patch image 85 in which the tissue specimen in which the morphological abnormality has occurred is imaged is acquired from, for example, a past dose group composed of a plurality of subjects S to which the candidate substance 27 is administered in the past evaluation test. As a result, the autoencoder 90 and the extraction model 80 can be trained for the tissue specimen having a more diverse feature of the shape and the texture. As a result, the extraction model 80 can extract the feature amount 87 that better represents the feature of the shape and the texture of the tissue specimen.

The patch image 85 in which the tissue specimen in which the morphological abnormality has occurred is imaged is not limited to the one acquired from the subject S constituting the past dose group illustrated as an example. In the subject S constituting the past control group 26P, the morphological abnormality may also occur. Therefore, in a case where the patch image 85 in which the tissue specimen in which the morphological abnormality has occurred is imaged, it does not matter whether the subject S is the past control group 26P or the past dose group. Further, the patch image 85 in which the tissue specimen in which the morphological abnormality has occurred is imaged may be an image acquired from the subject S that is designed to develop the morphological abnormality by applying various stresses. In addition, the patch image 85 in which the tissue specimen in which the morphological abnormality has occurred is imaged may be an image artificially created by processing the patch image 85 in which the normal tissue specimen is imaged.

An encoder unit of a convolutional neural network that outputs a class discrimination result in response to the input of the patch image 85 may be used as the extraction model 80 instead of the encoder unit 91 of the autoencoder 90. The class discrimination result is, for example, a result of discriminating one type of morphological abnormality that has occurred in the tissue specimen imaged in the patch image 85 from among a plurality of types such as hyperplasia, infiltration, congestion, and inflammation.

In addition, the machine learning model to be used as the extraction model 80 is not limited to the autoencoder 90 and the convolutional neural network illustrated as an example. A generator of a generative adversarial network (GAN) may be used as the extraction model 80. A machine learning model that does not have a convolutional layer, such as a vision transformer (ViT), may be used as the extraction model 80.

Contrastive learning of bringing the distance between the feature amounts derived from the same image closer to each other in the feature amount space and moving the distance between the feature amounts derived from different images farther from each other in the feature amount space may be performed. As the contrastive learning, for example, a learning method such as a simple framework for contrastive learning of visual representations (SimCLR) is known. In addition, a learning method such as bootstrap your own latent (BYOL) that does not use the above-described pair of different images (also referred to as a negative sample) may be used. In addition, a constraint such as a distribution on a unit sphere or a distribution following a standard normal distribution may be applied to the distribution of the feature amount to be extracted.

The feature amount 87 is not limited to the feature amount extracted by the extraction model 80. The feature amount 87 may be, for example, the average value, maximum value, minimum value, mode value, or variance of the pixel values of the patch image 85.

In each of the above-described embodiments, a case where one slide specimen 18 has a plurality of tissue specimens has been illustrated as an example, but the present disclosure is not limited to this. The technology of the present disclosure can also be applied to a case where one slide specimen 18 has one tissue specimen.

The subject S is not limited to the rat. The subject S may be a mouse, a guinea pig, a sand mouse, a hamster, a ferret, a rabbit, a dog, a cat, a monkey, or the like. In addition, the subject S may be a human.

The evaluation support apparatus 10 may be a personal computer that is installed in a pharmaceutical facility as shown in FIG. 1 or may be a server computer that is installed in a data center independent of the pharmaceutical facility.

In a case where the evaluation support apparatus 10 is configured by the server computer, the specimen image 15 is transmitted from the personal computer installed in each pharmaceutical facility to the server computer via a network such as the Internet. The server computer distributes various screens, such as the image list display screen 120, to the personal computer, for example, in a format of screen data for web distribution created by a markup language such as extensible markup language (XML). The personal computer reproduces a screen displayed on a web browser based on the screen data and displays the reproduced screen on the display. Note that, instead of XML, another data description language, such as JavaScript (registered trademark) Object Notation (JSON), may be used.

The evaluation support apparatus 10 according to the technology of the present disclosure can be widely used in all stages of pharmaceutical development from the setting of a drug discovery target in the earliest stage to the clinical trial in the final stage.

The hardware configuration of the computer constituting the evaluation support apparatus 10 according to the technology of the present disclosure can be modified in various ways. For example, the evaluation support apparatus 10 may be configured by a plurality of computers that are separated as hardware for the purpose of improving processing capacity and reliability. For example, the functions of the identification unit 51 and the first determination unit 52 and the functions of the extraction unit 53, the second determination unit 54, and the image processing unit 55 are distributed to two computers. In this case, the evaluation support apparatus 10 is configured by the two computers.

As described above, the hardware configuration of the computer of the evaluation support apparatus 10 can be changed as appropriate depending on required performance such as processing capacity, safety, and reliability. Further, it goes without saying that, in addition to the hardware, an application program such as the operation program 40 can be duplicated or distributed and stored in a plurality of storages for the purpose of ensuring the safety and the reliability.

In each of the above-described embodiments, for example, the following various processors described below can be used as a hardware structure of processing units that execute various types of processing, such as the RW control unit 50, the identification unit 51, the first determination unit 52, the extraction unit 53, the second determination unit 54, the image processing unit 55, and the display control unit 56. The various processors include, for example, in addition to the CPU 32 that is a general-purpose processor executing software (operation program 40) to function as various processing units as described above, a programmable logic device (PLD) that is a processor of which a circuit configuration can be changed after manufacture, such as a field programmable gate array (FPGA), and a dedicated electric circuit that is a processor having a dedicated circuit configuration designed to execute a specific process, such as an application specific integrated circuit (ASIC).

One processing unit may be configured by one of the various types of processors or may be configured by a combination of two or more processors of the same type or different types (for example, a combination of a plurality of FPGAs and/or a combination of a CPU and an FPGA). In addition, a plurality of processing units may be configured by one processor.

As an example of configuring the plurality of processing units with one processor, first, there is a form in which one processor is configured by a combination of one or more CPUs and software and the processor functions as the plurality of processing units, as represented by computers such as a client and a server. A second example of the configuration is a form in which a processor that implements the functions of the entire system including the plurality of processing units using one integrated circuit (IC) chip is used, as represented by a system on chip (SoC). As described above, the various processing units are configured by using one or more of the above various processors as the hardware structure.

In addition, more specifically, an electric circuit (circuitry) in which circuit elements, such as semiconductor elements, are combined can be used as the hardware structure of these various processors.

It is possible to understand the technology described in the following supplementary notes from the above description.

### [Supplementary Note 1]

An image processing apparatus comprising:
a processor,
in which the processor is configured to:
   acquire a specimen image in which a tissue specimen of a subject provided for an evaluation test of a candidate substance of a drug is imaged; and
   perform image processing on the specimen image in accordance with a type of a source organ of the tissue specimen and/or a type of a morphological abnormality estimated to have occurred in the tissue specimen.

### [Supplementary Note 2]

The image processing apparatus according to Supplementary Note 1,
in which the image processing is image processing that simulates a change in image quality of the specimen image due to a change in setting of an optical system in an imaging apparatus.

### [Supplementary Note 3]

The image processing apparatus according to Supplementary Note 2,
in which the image processing includes at least one of processing of adjusting brightness, processing of adjusting contrast, or enlargement processing.

### [Supplementary Note 4]

The image processing apparatus according to any one of Supplementary Notes 1 to 3,
in which the processor is configured to perform the image processing only on a region in which the morphological abnormality is estimated to have occurred.

### [Supplementary Note 5]

The image processing apparatus according to any one of Supplementary Notes 1 to 4,
in which the processor is configured to determine the type of the source organ of the tissue specimen and/or the type of the morphological abnormality.

### [Supplementary Note 6]

The image processing apparatus according to any one of Supplementary Notes 1 to 5,
in which the processor is configured to receive an instruction to perform the image processing from a user.

### [Supplementary Note 7]

The image processing apparatus according to any one of Supplementary Notes 1 to 6,
in which the processor is configured to extract a region in which the morphological abnormality is estimated to have occurred from the specimen image.

### [Supplementary Note 8]

The image processing apparatus according to Supplementary Note 7,
in which the processor is configured to perform the extraction by using a machine learning model.

### [Supplementary Note 9]

The image processing apparatus according to Supplementary Note 8,
in which the processor is configured to perform the extraction by comparing a feature amount obtained by inputting the specimen image to the machine learning model with a reference feature amount obtained by inputting a reference specimen image in which a tissue specimen considered to be normal is imaged to the machine learning model.

### [Supplementary Note 10]

The image processing apparatus according to any one of Supplementary Notes 7 to 9,
in which the processor is configured to:
classify the region in which the morphological abnormality is estimated to have occurred into a plurality of groups based on similarity of the morphological abnormality; and
perform the same image processing on a group basis.

The above various embodiments and/or various modification examples can be combined as appropriate in the technology of the present disclosure. In addition, it goes without saying that the present disclosure is not limited to each of the embodiments described above, and various configurations can be adopted without departing from the gist. Furthermore, the technology of the present disclosure extends to a storage medium that non-transitorily stores the program, and a computer program product including the program, in addition to the program.

The above description content and illustrated content are detailed descriptions of portions related to the technology of the present disclosure and are merely examples of the technology of the present disclosure. For example, the above description of the configurations, functions, operations, and effects is the description of examples of the configurations, functions, operations, and effects of portions according to the technology of the present disclosure. Therefore, it is needless to say that unnecessary portions may be deleted or new elements may be added or replaced in the above description content and illustrated content without departing from the gist of the technology of the present disclosure. In addition, in the above description content and illustrated content, the description of, for example, common technical knowledge that does not need to be particularly described to enable the implementation of the technology of the present disclosure is omitted in order to avoid confusion and facilitate the understanding of portions related to the technology of the present disclosure.

In the present specification, "A and/or B" is synonymous with "at least one of A or B". That is, "A and/or B" may mean only A, only B, or a combination of A and B. Further, in the present specification, the same concept as "A and/or B" is also applied to a case where three or more matters are linked and expressed by "and/or".

All documents, patent applications, and technical standards described in the present specification are incorporated in the present specification by reference to the same extent as in a case where each of the documents, patent applications, and technical standards are specifically and individually indicated to be incorporated by reference.

## Claims

1. An image processing apparatus comprising:
a processor,
wherein the processor is configured to:
acquire a specimen image in which a tissue specimen of a subject provided for an evaluation test of a candidate substance of a drug is imaged; and
perform image processing on the specimen image in accordance with a type of a source organ of the tissue specimen and/or a type of a morphological abnormality estimated to have occurred in the tissue specimen.

2. The image processing apparatus according to claim 1,
wherein the image processing is image processing that simulates a change in image quality of the specimen image due to a change in setting of an optical system in an imaging apparatus.

3. The image processing apparatus according to claim 2,
wherein the image processing includes at least one of processing of adjusting brightness, processing of adjusting contrast, or enlargement processing.

4. The image processing apparatus according to claim 1,
wherein the processor is configured to perform the image processing only on a region in which the morphological abnormality is estimated to have occurred.

5. The image processing apparatus according to claim 1,
wherein the processor is configured to determine the type of the source organ of the tissue specimen and/or the type of the morphological abnormality.

6. The image processing apparatus according to claim 1,
wherein the processor is configured to receive an instruction to perform the image processing from a user.

7. The image processing apparatus according to claim 1,
wherein the processor is configured to extract a region in which the morphological abnormality is estimated to have occurred from the specimen image.

8. The image processing apparatus according to claim 7,
wherein the processor is configured to perform the extraction by using a machine learning model.

9. The image processing apparatus according to claim 8,
wherein the processor is configured to perform the extraction by comparing a feature amount obtained by inputting the specimen image to the machine learning model with a reference feature amount obtained by inputting a reference specimen image in which a tissue specimen considered to be normal is imaged to the machine learning model.

10. The image processing apparatus according to claim 7,
wherein the processor is configured to:
classify the region in which the morphological abnormality is estimated to have occurred into a plurality of groups based on similarity of the morphological abnormality; and
perform the same image processing on a group basis.

11. An operation method of an image processing apparatus, the operation method comprising:
acquiring a specimen image in which a tissue specimen of a subject provided for an evaluation test of a candidate substance of a drug is imaged; and
performing image processing on the specimen image in accordance with a type of a source organ of the tissue specimen and/or a type of a morphological abnormality estimated to have occurred in the tissue specimen.

12. An operation program of an image processing apparatus, the operation program causing a computer to execute a process comprising:
acquiring a specimen image in which a tissue specimen of a subject provided for an evaluation test of a candidate substance of a drug is imaged; and
performing image processing on the specimen image in accordance with a type of a source organ of the tissue specimen and/or a type of a morphological abnormality estimated to have occurred in the tissue specimen.
